# EUROPÄISCHE PATENTSCHRIFT

(11) **EP 3 839 035 B1**
(45) Veröffentlichungstag und Bekanntmachung des Hinweises auf die Patenterteilung: **17.06.2026**
(21) Anmeldenummer: 19217334.2
(22) Anmeldetag: 18.12.2019
(51) Int. Cl.: C12M 1/24, C12M 1/00, C12M 1/06, C12M 1/34, C12M 1/36, G05B 19/042

(54) **BIOREAKTOR, KOPFPLATTE FÜR EINEN BIOREAKTOR UND DATENÜBERTRAGUNGSEINHEIT FÜR EINE KOPFPLATTE**
BIOREACTOR, HEAD PLATE FOR A BIOREACTOR AND DATA TRANSMISSION UNIT FOR A HEAD PLATE
BIORÉACTEUR, PLAQUE DE TÊTE POUR UN BIORÉACTEUR ET UNITÉ DE TRANSFERT DES DONNÉES POUR UNE PLAQUE DE TÊTE

(43) Veröffentlichungstag der Anmeldung: 23.06.2021
(73) Patentinhaber: Eppendorf SE, 22339 Hamburg (DE)
(72) Erfinder: ERTEL, Guido, 41539 Dormagen (DE)
(74) Vertreter: RGTH

(56) Entgegenhaltungen:
- EP-A1- 2 674 479
- EP-A2- 1 653 306
- DE-A1- 102010 029 953
- DE-A1- 102016 103 456
- DE-A1- 102017 114 851
- US-A1- 2016 333 303

## Beschreibung

Die Erfindung betrifft einen Bioreaktor, insbesondere zur Kultivierung von Mikroorganismen und/oder Zellkulturen, eine Kopfplatte zum Verschließen eines derartigen Bioreaktors und eine Datenübertragungseinheit für diese Kopfplatte. Ferner betrifft die Erfindung ein Verfahren zum Bereitstellen einer Datenübertragungseinheit.

Bioreaktoren, häufig auch als Fermenter bezeichnet, sind Behälter, in denen bestimmte Mikroorganismen, Zellen oder Pflanzen, vorzugsweise unter möglichst optimalen, kontrollierten und reproduzierbaren Bedingungen, kultiviert werden, um Zellen selbst oder Teile von ihnen oder eines ihrer Stoffwechselprodukte zu gewinnen. Hierzu umschließen Bioreaktoren einen Reaktionsraum, in dem ein Kulturmedium aufgenommen werden kann.

Ein Bioreaktor kann im Labormaßstab, insbesondere im Screening, ein Volumen von wenigen Millilitern bis hin zu mehreren tausend Kubikmetern im Produktionsmaßstab aufweisen. Im Labormaßstab, insbesondere zu Zwecken der Forschung und Entwicklung, werden vorzugsweise Bioreaktoren mit einem Volumen bis zu zehn Liter eingesetzt. Die Bioreaktoren im Labormaßstab unterscheiden sich hinsichtlich eines Leistungseintrags und eines Masse- und Stofftransports sowie verwendeter Materialien von einem Bioreaktor im Produktionsmaßstab. Bioreaktoren im Labormaßstab sind oftmals aus Glas und/oder Metall, insbesondere rostfreiem Stahl, ausgebildet, da die Bioreaktoren zwischen verschiedenen Anwendungen sterilisiert, vorzugsweise durch Heißdampfsterilisation in einem Autoklav, werden müssen. Alternativ zu wiederverwendbaren Bioreaktoren können auch Einwegbioreaktoren zum Einsatz kommen. Einwegbioreaktoren werden für die Durchführung eines einzigen biologischen bzw. biotechnologischen Prozess verwendet und anschließend entsorgt. Durch die Bereitstellung eines neuen, vorzugsweise im Herstellungsprozess sterilisierten, Einwegbioreaktors für jeden einzelnen Prozess kann die Gefahr einer Kontamination reduziert werden und gleichzeitig entfällt der Aufwand der Durchführung eines Reinigungs- und Sterilisationsprozesses.

Bioreaktoren weisen meistens ein Rührwerk auf, dessen Rührwelle von einer Antriebseinheit in Rotation versetzt werden kann. Dadurch kann ein mit der Rührwelle drehsteif verbundenes Rührelement ebenfalls in Rotation versetzt und eine Durchmischung der im Reaktionsraum vorhandenen Stoffe, insbesondere des Kulturmediums, erzielt werden. Insbesondere können auch zwei oder mehrere Rührelemente, vorzugsweise axial beabstandet, auf der Rührwelle angeordnet und mit dieser verbunden sein. Das Rührelement kann bzw. die Rührelemente können auch einstückig mit der Rührwelle ausgebildet sein.

Darüber hinaus verfügen Bioreaktoren oftmals über mehrere Anschlüsse, Primär- und Sekundärstoffe sowie verschiedene Instrumente, wie zum Beispiel Sensoren und/oder Aktoren, die in den Reaktionsraum eingebracht werden können. Vorzugsweise können über diese Anschlüsse auch Fluidleitungen, insbesondere Gasleitungen, wie Begasungs- oder Abgasleitungen, angeschlossen werden.

Mithilfe der Sensoren und/oder der Aktoren kann eine optimale Produktausbeute angestrebt werden. Hierzu werden in der Regel Sensoren verwendet, die die Bedingungen im Reaktionsraum, insbesondere einen pH-Wert, ein Redoxpotential, einen O₂-Gehalt in dem Kulturmedium und gegebenenfalls der Abluft, einen CO₂-Gehalt in der Abluft, eine Temperatur, eine optische Dichte des Kulturmediums, eine Schaumentwicklung, einen Glucosegehalt, einen Glyceringehalt und/oder einen Lactatgehalt, überwachen. Ferner können verschiedene Aktoren, wie zum Beispiel Peltierkühler und/oder Leuchtstäbe, vorgesehen sein, um möglichst optimale Kultivierungsbedingungen zu schaffen.

Die Sensoren und die Aktoren - sofern vorhanden - werden über jeweils ein Kabel mit einem entsprechenden Messmodul und/oder einer Auswerteeinheit und/oder einer Steuereinheit verbunden. Diese Kabel sind in der Regel gut geschirmt und speziell aufgebaut, um die Integrität der Daten zu sichern, die durch äußere Einflüsse, wie zum Beispiel elektromagnetische Felder, erheblich gestört werden kann. Die Anzahl der Kabel bei mehreren Sensoren und/oder Aktoren kann zu einer hohen Unübersichtlichkeit führen.

Ferner schränkt die Anzahl der Kabel die Handhabung des Bioreaktors ein. Insbesondere bei Bioreaktoren, die einen kleineren Maßstab aufweisen, zum Beispiel im 300 ml Bereich, ist die Einschränkung in der Handhabung besonders spürbar.

DE102010029953 betrifft ein Verfahren unter anderem zum Betreiben und/oder Bedienen von Feldgeräten. Die Feldgeräte sind hierbei über zu- mindest einen Datenbus miteinander verbunden. Der Zugriff auf die Feld- geräte erfolgt über eine Feldgerätzugriffseinheit, die mit zumindest einer Automatisierungs-/Integrations-Plattform und zu- mindest einem zentralen WebServer eines Service Providers kommuniziert.

DE102017114851 betrifft einen Feldgerätadapter zur drahtlosen Daten- übertragung. Der Adapter umfasst hierbei ein Adaptergehäuse mit einem ersten und einem zweiten Ende. Das erste Ende ist ausgebildet, um den Feldgeräteadapter an ein Feldgerät mechanisch anzuschließen.

EP2674479 offenbart einen Einweg-Bioreaktor mit Kopfplatte.

Der Erfindung liegt daher die Aufgabe zugrunde, eine verbesserte Lösung bereitzustellen, welche wenigstens eines der genannten Probleme adressiert. Insbesondere ist es eine Aufgabe der Erfindung, eine Lösung bereitzustellen, die hinsichtlich der Handhabung des Bioreaktors verbessert ist und gleichzeitig eine robuste Datenübertragung ermöglicht.

Gemäß einem ersten Aspekt wird die eingangs genannte Aufgabe gelöst durch einen Bioreaktor, insbesondere zur Kultivierung von Mikroorganismen und/oder Zellkulturen, mit einem Behälter, einer Kopfplatte, die ausgebildet ist, um den Behälter zu verschließen, einem Rührwerk mit einer durch die Kopfplatte, in einen Innenraum des Behälters geführten Rührwelle und einem in dem Innenraum des Behälters mit der Rührwelle verbundenen Rührelement, einer an der Kopfplatte angeordneten, mit der Rührwelle gekoppelten Antriebseinheit und mindestens einem in dem Innenraum angeordneten Feldgerät mit einer Feldgerätelektronik, wobei die Kopfplatte umfasst: eine Innenseite und eine der Innenseite gegenüberliegende Außenseite, eine Rührwerksanschlusseinrichtung, die ausgebildet ist, um die Rührwelle durch die Kopfplatte hindurchzuführen und die Antriebseinheit anzuschließen, mindestens eine Feldgerätanschlusseinrichtung, die ausgebildet ist, um das Feldgerät an der Innenseite anzuschließen, und eine an der Außenseite angeordnete Datenübertragungseinheit, mit einem Motorkopfstück, das mit einer Auswerteeinheit signaltechnisch koppelbar und mit der Antriebseinheit energietechnisch gekoppelt ist, umfassend eine Motorkopfelektronik, und mindestens einer mit dem Motorkopfstück signaltechnisch gekoppelten Kommunikationseinheit, umfassend: ein Adapterstück mit einer Adapterelektronik, die mit der Feldgerätelektronik des Feldgeräts elektrisch verbunden ist, und ein Kommunikatorstück mit einer Kommunikatorelektronik, die mit der Adapterelektronik und der Motorkopfelektronik elektrisch verbunden ist, wobei die Adapterelektronik eingerichtet ist, um Signale zwischen der Feldgerätelektronik und der Kommunikatorelektronik zu kommunizieren, wobei die Signale Feldgerätinformationen und Messinformationen und/oder Steuerinformation tragen, wobei die Kommunikatorelektronik eingerichtet ist, um die Signale zwischen der Adapterelektronik und der Motorkopfelektronik zu kommunizieren, wobei die Datenübertragungseinheit ferner einen Signalwandler umfasst, der vorzugsweise als Teil der Adapterelektronik und/oder als Teil der Kommunikatorelektronik ausgebildet ist, wobei das Adapterstück und das Kommunikatorstück als separate, miteinander koppelbare Bauteile ausgebildet sind, wobei das Motorkopfstück als ein separates, mit der Kommunikationseinheit koppelbares Bauteil ausgebildet ist.

In einer bevorzugten Ausführungsform ist ein Bioreaktor, insbesondere zur Kultivierung von Mikroorganismen und/oder Zellkulturen, vorgesehen mit einem Behälter, einer Kopfplatte, die ausgebildet ist, um den Behälter zu verschließen, einem Rührwerk mit einer durch die Kopfplatte, in einen Innenraum des Behälters geführten Rührwelle und einem in dem Innenraum des Behälters mit der Rührwelle verbundenen Rührelement, einer an der Kopfplatte angeordneten, mit der Rührwelle gekoppelten Antriebseinheit und mindestens zwei in dem Innenraum angeordneten Feldgeräten mit jeweils einer Feldgerätelektronik, wobei die Kopfplatte umfasst: eine Innenseite und eine der Innenseite gegenüberliegende Außenseite, eine Rührwerksanschlusseinrichtung, die ausgebildet ist, um die Rührwelle durch die Kopfplatte hindurchzuführen und die Antriebseinheit anzuschließen, mindestens zwei Feldgerätanschlusseinrichtungen, die ausgebildet sind, um jeweils eines der Feldgeräte an der Innenseite anzuschließen, und eine an der Außenseite angeordnete Datenübertragungseinheit, mit einem Motorkopfstück, das mit einer Auswerteeinheit signaltechnisch koppelbar und mit der Antriebseinheit energietechnisch gekoppelt ist, umfassend eine Motorkopfelektronik, und mindestens zwei, jeweils mit dem Motorkopfstück signaltechnisch gekoppelten Kommunikationseinheiten, jeweils umfassend: ein Adapterstück mit einer Adapterelektronik, die mit der Feldgerätelektronik eines der Feldgeräte elektrisch verbunden ist, und ein Kommunikatorstück mit einer Kommunikatorelektronik, die mit der Adapterelektronik und der Motorkopfelektronik elektrisch verbunden ist, wobei die Adapterelektronik eingerichtet ist, um Signale zwischen der Feldgerätelektronik und der Kommunikatorelektronik zu kommunizieren, wobei die Signale Feldgerätinformationen und Messinformationen und/oder Steuerinformation tragen, wobei die Kommunikatorelektronik eingerichtet ist, um die Signale zwischen der Adapterelektronik und der Motorkopfelektronik zu kommunizieren, wobei die Datenübertragungseinheit ferner einen Signalwandler umfasst, der vorzugsweise als Teil der Adapterelektronik und/oder als Teil der Kommunikatorelektronik ausgebildet ist, wobei das Adapterstück und das Kommunikatorstück als separate, miteinander koppelbare Bauteile ausgebildet sind, wobei das Motorkopfstück als ein separates, mit den Kommunikationseinheiten koppelbares Bauteil ausgebildet ist.

In der hier beschriebenen Lösung wird der Bioreaktor mit der Kopfplatte und der an der Außenseite der Kopfplatte angeordneten Datenübertragungseinheit, die eine Signalübertragung zwischen dem Feldgerat/den Feldgeräten und der Auswerteeinheit ermöglicht, bereitgestellt. Ein derartiger Bioreaktor umfasst den Behälter, der durch die Kopfplatte verschlossen werden kann und dessen Innenraum vorzugsweise einen Reaktionsraum definiert. Der Bioreaktor umfasst ferner das Rührwerk, das mittels der Antriebseinheit angetrieben werden kann, insbesondere um ein Kulturmedium innerhalb des Reaktionsraums zu durchmischen. Zur Überwachung und/oder Einstellung angestrebter Reaktionsbedingungen umfasst der Bioreaktor ferner ein Feldgerät oder zwei oder mehrere Feldgeräte, insbesondere Sensoren und/oder Aktoren, das/die in dem Innenraum angeordnet ist/sind. Das Feldgerät/die Feldgeräte ist/sind hierbei an den Feldgerätanschlusseinrichtungen der Kopfplatte angeordnet. Die Datenübertragungseinheit ist an der Außenseite der Kopfplatte angeordnet und ausgebildet, um zwischen dem Feldgerät/den Feldgeräten und der Auswerteeinheit zu kommunizieren. Die Datenübertragungseinheit umfasst den Motorkopf, das mindestens eine Adapterstück und das mindestens eine Kommunikatorstück. Vorzugsweise kann der Motorkopf auf der Antriebseinheit montiert sein.

Der Bioreaktor, insbesondere die Kopfplatte, kann vorzugsweise mit unterschiedlichen Feldgeräten, insbesondere mit unterschiedlichen Sensoren und/oder Aktoren ausgestattet werden. Ein Feldgerät kann beispielsweise als Sensor, Aktor oder sonstige Komponente eines Bioreaktors ausgebildet sein. Bevorzugt kann der Bioreaktor Feldgeräte umfassen, um Parameter, die vorzugsweise während einer Kultivierung von bakteriellen, tierischen und humanen Zellen erfasst werden können, zu überwachen. Derartige Parameter können vorzugsweise Temperatur, pH-Wert, Gelöstsauerstoffgehalt sowie Ist-Werte von Aktoren, wie zum Beispiel Pumpen, Rührer, Peltierkühler, Leuchtstäbe und/oder Begasung sein. Darüber hinaus können weitere Feldgeräte vorgesehen sein, um Daten wie zum Beispiel Lebendzelldichte, Nährstoffkonzentration und/oder Metabolitkonzentration zu ermitteln. Ein als Sensor ausgebildetes Feldgerät kann beispielsweise als pH-Sensor, RedoxPotential Sensor, Temperatursensor, Gelöstsauerstoffgehalt-Sensor etc ausgebildet sein. Ein als Aktor ausgebildetes Feldgerät kann beispielsweise als Pumpe, Rührer, Peltierkühler und/oder Leuchtstab etc ausgebildet sein.

Entsprechend der Feldgeräte kann ein geeignetes Adapterstück ausgewählt und/oder hergestellt werden, dessen Adapterstückelektronik ausgebildet ist, um Signale von dem Feldgerät zu empfangen und/oder Signale an dieses Feldgerät zu senden.

Die Kommunikatorelektronik eines Kommunikatorstücks ist mit der Motorkopfelektronik elektrisch verbunden, um Signale eines Feldgeräts, insbesondere über ein Adapterstück, zu empfangen und diese an die Motorkopfelektronik zu senden und/oder Signale von der Motorkopfelektronik zu empfangen und, insbesondere über ein Adapterstück, an das Feldgerät zu senden. Dadurch kann die Motorkopfelektronik Signale der einzelnen Feldgeräte empfangen und/oder Signale an diese senden.

Der Signalwandler ist ausgebildet, um die Signale umzusetzen. Der Signalwandler kann vorzugsweise als Teil der Adapterelektronik und/oder als Teil der Kommunikatorelektronik ausgebildet sein und/oder sowohl die Adapterelektronik als auch die Kommunikatorelektronik kann jeweils einen Signalwandler umfassen.

Analoge Signale eines Feldgeräts werden vorzugsweise zunächst in normierte Signale (zum Beispiel 0 V bis 4,095 V) umgewandelt und dann in digitale Signale (zum Beispiel 0 bis 4095) umgesetzt. Diese Umwandlung bzw. Umsetzung erfolgt vorzugsweise in der Kommunikationseinheit, insbesondere in der Adapterelektronik und/oder in der Kommunikatorelektronik. Vorzugsweise kann die Adapterstückelektronik ausgebildet sein, um Signale von der Kommunikatorelektronik zu empfangen, mittels des Signalwandlers umzusetzen bzw. umzuwandeln und an die Adapterelektronik zu senden. Beispielsweise kann die Umwandlung bzw. Umsetzung der Signale vollständig in der Adapterelektronik erfolgen. Die Umwandlung bzw. Umsetzung der Signale kann auch vollständig in der Kommunikatorelektronik erfolgen. Die Umwandlung bzw. Umsetzung der Signale auch teilweise in der Adapterelektronik und teilweise in der Kommunikatorelektronik erfolgen. Beispielsweise kann in der Adapterelektronik eine Umsetzung analoger Signale eines Feldgeräts in analoge normierte Signale erfolgen und die Umsetzung der analogen normierten Signale in digitale Signale kann in der Kommunikatorelektronik erfolgen.

Durch unterschiedliche Adapterstücke, die entsprechend der Feldgeräte konfiguriert sind, kann der Bioreaktor individuell an Kundenbedürfnisse angepasst werden. Besonders bevorzugt kann der Bioreaktor individuell für Kundenbedürfnisse hergestellt werden.

Durch diese Ausgestaltung kann ein besonders vorteilhafter Aufbau des Bioreaktors erzielt werden. Insbesondere kann eine Kabelage an dem Bioreaktor reduziert werden. Das führt insgesamt zu aufgeräumteren Laborarbeitsplätzen und einer einfacheren Handhabung des Bioreaktors.

Es sind auch Ausgestaltungen möglich, bei denen für zwei oder mehr Feldgeräte mit jeweils einem zugehörigen Adapterstück ein gemeinsames Kommunikatorstück vorgesehen ist. In einer solchen Ausgestaltung ist dann vorzugsweise das Kommunikatorstück, insbesondere die Kommunikatorelektronik, ausgebildet, mit der Adapterelektronik von zwei oder mehr Adapterstücken zu kommunizieren.

Vorzugsweise kann die Antriebseinheit eine Antriebselektronik umfassen, die ausgebildet ist, um mit der Motorkopfelektronik zu kommunizieren. Die Antriebselektronik und die Motorkopfelektronik können vorzugweise kabellos, insbesondere über Funk, oder über ein Kabel elektrisch miteinander verbunden sein. Insbesondere kann die Energieversorgung kontaktgebunden, vorzugsweise kabelgebunden, oder kabellos, insbesondere induktiv, erfolgen. Die bevorzugte induktive Energieversorgung kann vorzugsweise von einer induktiven Quelle im Motorkopfstück gespeist sein.

Vorzugsweise ist die Antriebselektronik ausgebildet, die Antriebseinheit anzusteuern. Die Antriebselektronik kann vorzugsweise räumlich getrennt von der Antriebseinheit angeordnet sein. Beispielsweise kann die Antriebselektronik zusammen mit der Auswerteeinheit und/oder mit einer Steuereinheit angeordnet sein.

Der Motorkopf kann beispielsweise energietechnisch mit der Antriebseinheit gekoppelt sein, um diese mit Energie zu versorgen. Die Antriebseinheit kann auch unabhängig vom Motorkopf mit Energie versorgt werden.

Das Rührwerk umfasst die Rührwelle, die durch die Kopfplatte in den Innenraum hindurchgeführt ist und Rührelemente aufweist. Die Rührwelle ist mit der an der Kopfplatte angeordneten Antriebseinheit gekoppelt. Die Antriebseinheit kann vorzugsweise an einem oberen Ende der Rührwelle angreifen. Die Rührwelle kann vorzugsweise in einem Lager um eine Rotationsachse drehbar gelagert sein. Insbesondere können die Rührelemente an der Rührwelle drehsteif befestigt sein.

Die Rührwelle kann vorzugsweise im Wesentlichen vertikal angeordnet sein und mit den Rührelementen das Rührwerk bilden. Die Rührelemente können sich vorzugweise jeweils in horizontaler Ebene bewegen. Insbesondere bevorzugt können die Rührelemente eine Neigung gegenüber der Vertikalen, insbesondere gegenüber der Rührwelle, aufweisen. Vorzugsweise können die Rührelemente unterschiedliche Neigungen gegenüber der Vertikalen, insbesondere gegenüber der Rührwelle, aufweisen. Vorzugsweise kann der Bioreaktor auch zwei oder mehrere Rührwerke aufweisen.

Der Behälter kann vorzugsweise aus Kunststoff oder Glas ausgebildet sein. Insbesondere bevorzugt kann der Behälter formstabil ausgebildet sein. Der Behälter kann vorzugsweise einen Boden und eine den Boden umschließende Wandung aufweisen. Durch diese Ausgestaltung kann der Behälter gereinigt und sterilisiert werden, um wieder verwendet werden zu können.

Der Bioreaktor kann vorzugsweise auch als ein Einweg-Bioreaktor ausgebildet sein. Der Behälter kann insbesondere flexibel, vorzugsweise als ein Beutel, ausgebildet sein. Durch diese Ausgestaltung kann die Notwendigkeit aufwändiger Sterilisations- und Reinigungsprozesse vermieden werden. Ferner kann das Risiko eines verfälschten oder unbrauchbaren Ergebnisses aufgrund einer nicht vollständigen Sterilisation und einer daraus folgenden Störung eines Prozesses verringert, insbesondere vermieden, werden.

Der Behälter kann vorzugsweise eine Eintragseinrichtung zum Zuführen von Eingangsstoffen und eine Entnahmeöffnung zur Entnahme eines Endprodukts und/oder einer Probe aufweisen. Vorzugsweise kann die Eintragseinrichtung in einem oberen Bereich des Behälters und die Entnahmeöffnung in einem unteren Bereich des Behälters angeordnet werden.

Besonders bevorzugt kann die Auswerteeinheit als eine Reaktorsteuerung ausgebildet sein. Vorzugsweise kann die Reaktorsteuerung die Daten der Feldgeräte sammeln, vorzugsweise visualisieren und vorzugsweise speichern. Diese Daten können vorzugsweise auch gemeinsam mit Prozessdaten in einer zentralen Datenbank gespeichert werden. Dadurch können die Daten insbesondere für eine Prozessentwicklung innerhalb der Reaktorsteuerung zur Verfügung stehen und können vorzugsweise für eine Kontrolle und/oder eine Prozessführung genutzt werden. Insbesondere können die Daten ausgewertet und genutzt werden, um Aktoren in Abhängigkeit der erfassten Daten anzusteuern.

Für weitere Vorteile, Ausführungsvarianten und Ausführungsdetails dieses ersten Aspekts und dessen mögliche Fortbildungen wird auch auf die im Folgenden ausgeführte Beschreibung zu den entsprechenden Merkmalen und Fortbildungen des zweiten Aspekts und des dritten Aspekts verwiesen.

Gemäß einem zweiten Aspekt wird die eingangs genannte Aufgabe gelöst durch eine Kopfplatte zum Verschließen eines Bioreaktors, umfassend eine Innenseite und eine der Innenseite gegenüberliegende Außenseite, eine Rührwerksanschlusseinrichtung, die ausgebildet ist, um eine Rührwelle durch die Kopfplatte hindurchzuführen und eine mit der Rührwelle koppelbare Antriebseinheit anzuschließen, mindestens eine Feldgerätanschlusseinrichtung, die ausgebildet ist, um ein Feldgerät an der Innenseite anzuschließen, eine an der Außenseite angeordnete Datenübertragungseinheit, mit einem Motorkopfstück, das mit einer Auswerteeinheit signaltechnisch und mit der Antriebseinheit, die an einer Kopfplatte eines Bioreaktors anordenbar ist, energietechnisch koppelbar ist, umfassend eine Motorkopfelektronik, und mindestens einer mit dem Motorkopfstück signaltechnisch gekoppelten Kommunikationseinheit, umfassend: ein Adapterstück mit einer Adapterelektronik, die mit einer Feldgerätelektronik des Feldgeräts elektrisch verbindbar ist, und ein Kommunikatorstück mit einer Kommunikatorelektronik, die mit der Adapterelektronik und der Motorkopfelektronik elektrisch verbunden ist, wobei die Adapterelektronik eingerichtet ist, um Signale zwischen der Feldgerätelektronik und der Kommunikatorelektronik zu kommunizieren, wobei die Signale Feldgerätinformationen und Messinformationen und/oder Steuerinformation tragen, wobei die Kommunikatorelektronik eingerichtet ist, um die Signale zwischen der Adapterelektronik und der Motorkopfelektronik zu kommunizieren, wobei die Datenübertragungseinheit ferner einen Signalwandler umfasst, der vorzugsweise als Teil der Adapterelektronik und/oder als Teil der Kommunikatorelektronik ausgebildet ist, wobei das Adapterstück und das Kommunikatorstück als separate, miteinander koppelbare Bauteile ausgebildet sind, wobei das Motorkopfstück als ein separates, mit der Kommunikationseinheit koppelbares Bauteil ausgebildet ist.

Vorzugsweise ist eine Kopfplatte zum Verschließen eines Bioreaktors vorgesehen, umfassend eine Innenseite und eine der Innenseite gegenüberliegende Außenseite, eine Rührwerksanschlusseinrichtung, die ausgebildet ist, um eine Rührwelle durch die Kopfplatte hindurchzuführen und eine mit der Rührwelle koppelbare Antriebseinheit anzuschließen, mindestens zwei Feldgerätanschlusseinrichtungen, die ausgebildet sind, um jeweils ein Feldgerät an der Innenseite anzuschließen, eine an der Außenseite angeordnete Datenübertragungseinheit, mit einem Motorkopfstück, das mit einer Auswerteeinheit signaltechnisch und mit der Antriebseinheit, die an einer Kopfplatte eines Bioreaktors anordenbar ist, energietechnisch koppelbar ist, umfassend eine Motorkopfelektronik, und mindestens zwei, jeweils mit dem Motorkopfstück signaltechnisch gekoppelten Kommunikationseinheiten, jeweils umfassend: ein Adapterstück mit einer Adapterelektronik, die mit einer Feldgerätelektronik eines der Feldgeräte elektrisch verbindbar ist, und ein Kommunikatorstück mit einer Kommunikatorelektronik, die mit der Adapterelektronik und der Motorkopfelektronik elektrisch verbunden ist, wobei die Adapterelektronik eingerichtet ist, um Signale zwischen der Feldgerätelektronik und der Kommunikatorelektronik zu kommunizieren, wobei die Signale Feldgerätinformationen und Messinformationen und/oder Steuerinformation tragen, wobei die Kommunikatorelektronik eingerichtet ist, um die Signale zwischen der Adapterelektronik und der Motorkopfelektronik zu kommunizieren, wobei das Adapterstück und das Kommunikatorstück als separate, miteinander koppelbare Bauteile ausgebildet sind, wobei die Datenübertragungseinheit ferner einen Signalwandler umfasst, der vorzugsweise als Teil der Adapterelektronik und/oder als Teil der Kommunikatorelektronik ausgebildet ist, wobei das Motorkopfstück als ein separates, mit den Kommunikationseinheiten koppelbares Bauteil ausgebildet ist.

In der hier beschriebenen Lösung wird die Kopfplatte zum Verschließen des Bioreaktors und der an der Außenseite der Kopfplatte angeordnete Datenübertragungseinheit, die eine Signalübertragung zwischen dem/den anschließbaren Feldgerät/Feldgeräten und der signaltechnisch koppelbaren Auswerteeinheit ermöglicht, bereitgestellt. Eine derartige Kopfplatte weist die Innenseite und die Außenseite auf. Die Kopfplatte kann vorzugsweise derart an einem Behälter des Bioreaktors angebracht und befestigt werden, dass die Innenseite einem Innenraum des Behälters zugewandt ist und den Innenraum begrenzt. Die Außenseite kann vorzugsweise der Innenseite gegenüberliegend ausgebildet sein und insbesondere dem Innenraum des Behälters abgewandt sein. Die Kopfplatte umfasst ferner die Rührwerksanschlusseinrichtung, um ein Rührwerk mit der Rührwelle an der Kopfplatte zu befestigen und die Antriebseinheit an der Rührwelle anzuschließen. Darüber hinaus weist die Kopfplatte Feldgerätanschlusseinrichtung(en) auf, um ein oder mehrere Feldgeräte, insbesondere Sensoren und/oder Aktoren, an dieser anzuschließen.

Die Feldgerätanschlusseinrichtungen können vorzugsweise sämtliche Einrichtungen zur Befestigung von Feldgeräten sein. Insbesondere können die Feldgeräte formschlüssig und/oder kraftschlüssig an diesen Feldgerätanschlusseinrichtungen befestigt werden.

Vorzugsweise kann eine Feldgerätanschlusseinrichtung eine Membran aufweisen, die den Innenraum abschließt, vorzugsweise abdichtet. Die Membran kann ausgebildet sein, um ein Feldgerät von außerhalb des Bioreaktors durch die Membran hindurchzuführen und in den Innenraum einzuführen. Durch die mit dem Vorsehen einer Membran erzielten Sterilität können Bioreaktoren besonders einfach mit Feldgeräten bedarfsorientiert ausgestattet und/oder aufgerüstet und/oder umgerüstet werden. Ferner können hierdurch Feldgeräte im Falle eines Defekts besonders einfach ausgetauscht werden.

Für weitere Vorteile, Ausführungsvarianten und Ausführungsdetails dieses zweiten Aspekts und dessen mögliche Fortbildungen wird auch auf die im Folgenden ausgeführte Beschreibung zu den entsprechenden Merkmalen und Fortbildungen des dritten Aspekts sowie auf die zuvor erfolgte Beschreibung zu den entsprechenden Merkmalen und Fortbildungen des ersten Aspekts verwiesen.

Gemäß einem dritten Aspekt wird die eingangs genannte Aufgabe gelöst durch eine Datenübertragungseinheit für eine Kopfplatte eines Bioreaktors mit einem Motorkopfstück, das mit einer Auswerteeinheit signaltechnisch und mit einer Antriebseinheit, die an einer Kopfplatte eines Bioreaktors anordenbar ist, energietechnisch koppelbar ist, umfassend eine Motorkopfelektronik, und mindestens einer mit dem Motorkopfstück signaltechnisch gekoppelten Kommunikationseinheit, umfassend: ein Adapterstück mit einer Adapterelektronik, die mit einer Feldgerätelektronik eines Feldgeräts elektrisch verbindbar ist, und ein Kommunikatorstück mit einer Kommunikatorelektronik, die mit der Adapterelektronik und der Motorkopfelektronik elektrisch verbunden ist, wobei die Adapterelektronik eingerichtet ist, um Signale zwischen der Feldgerätelektronik und der Kommunikatorelektronik zu kommunizieren, wobei die Signale Feldgerätinformationen und Messinformationen und/oder Steuerinformation tragen, wobei die Kommunikatorelektronik eingerichtet ist, um die Signale zwischen der Adapterelektronik und der Motorkopfelektronik zu kommunizieren, wobei die Datenübertragungseinheit ferner einen Signalwandler umfasst, der vorzugsweise als Teil der Adapterelektronik und/oder als Teil der Kommunikatorelektronik ausgebildet ist, wobei das Adapterstück und das Kommunikatorstück als separate, miteinander koppelbare Bauteile ausgebildet sind, wobei das Motorkopfstück als ein separates, mit der Kommunikationseinheit koppelbares Bauteil ausgebildet ist.

Vorzugsweise ist eine Datenübertragungseinheit für eine Kopfplatte eines Bioreaktors vorgesehen mit einem Motorkopfstück, das mit einer Auswerteeinheit signaltechnisch und mit einer Antriebseinheit, die an einer Kopfplatte eines Bioreaktors anordenbar ist, energietechnisch koppelbar ist, umfassend eine Motorkopfelektronik, und mindestens zwei, jeweils mit dem Motorkopfstück signaltechnisch gekoppelten Kommunikationseinheiten, jeweils umfassend: ein Adapterstück mit einer Adapterelektronik, die mit einer Feldgerätelektronik eines Feldgeräts elektrisch verbindbar ist, und ein Kommunikatorstück mit einer Kommunikatorelektronik, die mit der Adapterelektronik und der Motorkopfelektronik elektrisch verbunden ist, wobei die Adapterelektronik eingerichtet ist, um Signale zwischen der Feldgerätelektronik und der Kommunikatorelektronik zu kommunizieren, wobei die Signale Feldgerätinformationen und Messinformationen und/oder Steuerinformation tragen, wobei die Kommunikatorelektronik eingerichtet ist, um die Signale zwischen der Adapterelektronik und der Motorkopfelektronik zu kommunizieren, wobei die Datenübertragungseinheit ferner einen Signalwandler umfasst, der vorzugsweise als Teil der Adapterelektronik und/oder als Teil der Kommunikatorelektronik ausgebildet ist, wobei das Adapterstück und das Kommunikatorstück als separate, miteinander koppelbare Bauteile ausgebildet sind, wobei das Motorkopfstück als ein separates, mit den Kommunikationseinheiten koppelbares Bauteil ausgebildet ist.

Der Erfindung liegt unter anderem die Erkenntnis zugrunde, dass die Signale der Feldgeräte, insbesondere Sensorsignale, oftmals störanfällig sind. Meist kommen Feldgeräte zum Einsatz, die ausgebildet sind, um analoge Signale zu senden und/oder zu empfangen. Analoge Signale können besonders störanfällig sein. Aus diesem Grund kann insbesondere eine Übertragung dieser Signale mittels langer Kabel, vorzugsweise mittels Kabel mit einer Länge von mindestens 100 cm, nachteilig sein.

Ferner liegt der Erfindung die Erkenntnis zu Grunde, dass bei bekannten Lösungen jedes Feldgerät oftmals einzeln über ein Kabel mit einer Auswerteeinheit verbunden ist, um ein Signal zu übertragen. Kommen mehr als ein einziges Feldgerät zum Einsatz, kann die Kabelage der einzelnen Feldgeräte zu einer Unübersichtlichkeit führen und die Handhabbarkeit, insbesondere der Datenübertragungseinheit vorzugsweise im Rahmen einer Installation und/oder während eines Einsatzes, negativ beeinflussen. Zudem hat die direkte Verbindung eines Feldgeräts mit der Auswerteeinheit zur Folge, dass für jedes Feldgerät ein passendes Modul bzw. eine entsprechende Auswertelektronik entwickelt werden muss, um Signale der verschiedenen Feldgeräte empfangen und auswerten und/oder Signale an die verschiedenen Feldgeräte senden zu können. Aus diesem Grund muss bei bisher bekannten Lösungen bei Einsatz eines neuen Feldgeräts die entsprechende Auswerteelektronik bzw. ein passendes Modul entwickelt werden. Das kann oftmals zu längeren Wartezeiten bis zum Einsatz der neuen Feldgeräte führen.

In der hier beschriebenen Lösung wird eine Datenübertragungseinheit mit dem Motorkopfstück und mindestens einer Kommunikationseinheit, vorzugsweise mit mindestens zwei Kommunikationseinheiten bereitgestellt. Eine Kommunikationseinheit umfasst das Adapterstück und das Kommunikatorstück. Durch signaltechnisches Koppeln des Motorkopfstücks mit der Auswerteeinheit und jeweils des Adapterstücks mit dem Feldgerät kann mittels der Datenübertragungseinheit eine robuste Signalübertragung zwischen dem Feldgerät und der Auswerteeinheit ermöglicht werden.

In Abhängigkeit der Feldgeräte, die zum Einsatz kommen sollen, kann vorzugsweise für jedes Feldgerät ein speziell konfiguriertes Adapterstück ausgewählt und/oder hergestellt werden. Diese Adapterstücke umfassen jeweils eine Adapterelektronik, die ausgebildet ist um Signale von dem entsprechenden Feldgerät zu empfangen und/oder Signale an dieses Feldgerät zu senden.

Unterschiedliche Feldgeräte sind in der Regel eingerichtet, um unterschiedliche Signale, die Feldgerätinformationen und Messinformationen tragen, zu senden und/oder unterschiedliche Signale, die Steuerinformationen tragen, zu empfangen. Beispielsweise können Feldgeräte digitale oder analoge Signale senden und/oder empfangen. Insbesondere können Feldgeräte analoge Signale in einem bestimmten Frequenzbereich oder im Gleichstrombereich senden und/oder erzeugen. Aus diesem Grund ist es vorteilhaft entsprechend konfigurierte Adapterstücke einzusetzen, um diese Signale umsetzen und an das Kommunikatorstück senden zu können und/oder diese Signale von dem Kommunikatorstück empfangen, umsetzen und an das Feldgerät senden zu können.

Durch das Senden von Signalen, die Feldgerätinformationen und Messinformationen tragen, können Messdaten, die mithilfe des Feldgeräts erfasst wurden, und vorzugsweise Daten zu dem Feldgerät, wie zum Beispiel einer Information mittels welchem Feldgerät diese Messdaten erfasst werden, an das Kommunikatorstück übertragen werden. Die Feldgerätinformationen können vorzugsweise der Kommunikatorelektronik einen Feldgerättyp mitteilen.

Durch das Mitteilen des Feldgerättyps können Eigenschaften des Feldgeräts übermittelt werden. Diese Eigenschaften können eine Sensorart und/oder eine Aktorart und ob diese Signale digital oder analog übermitteln und/oder ähnliches umfassen.

Durch das Empfangen von Signalen, die Steuerinformationen tragen, und das Senden dieser Signale an das Feldgerät kann dieses angesteuert werden. Insbesondere kann dadurch das Feldgerät aktiviert und/oder deaktiviert und/oder eingestellt und/oder kalibriert werden.

Das Kommunikatorstück kann vorzugsweise im Gegensatz zu dem Adapterstück für jede Kommunikationseinheit gleich ausgebildet sein. Die Kommunikatorstücke können demnach vorzugsweise einheitlich konfiguriert sein. Die Kommunikatorelektronik jedes Kommunikatorstücks ist mit der Motorkopfelektronik elektrisch verbunden, um Signale des Feldgeräts über das Adapterstück zu empfangen und diese an die Motorkopfelektronik zu senden und/oder Signale von der Motorkopfelektronik zu empfangen und über das Adapterstück an das Feldgerät zu senden. Dadurch kann die Motorkopfelektronik Signale der einzelnen Feldgeräte empfangen und/oder Signale an diese senden.

Das Motorkopfstück ist hierbei mit der Auswerteeinheit zumindest signaltechnisch koppelbar. Insbesondere kann die Motorkopfelektronik mit einer Auswerteelektronik der Auswerteeinheit elektrisch verbunden werden. Vorzugsweise kann das Motorkopfstück ausgebildet sein, um mit der Auswerteeinheit energietechnisch gekoppelt zu werden. Dadurch kann die Auswerteeinheit die Motorkopfelektronik vorzugsweise auch mit Energie versorgen.

Das Motorkopfstück ist zumindest energietechnisch mit der Antriebseinheit verbunden und ausgebildet, um die Antriebseinheit mit Energie zu versorgen. Insbesondere kann die Motorkopfelektronik mit einer Antriebselektronik der Antriebseinheit elektrisch verbunden werden. Vorzugsweise kann das Motorkopfstück ausgebildet sein, um mit der Antriebseinheit signaltechnisch gekoppelt zu werden.

Unter einer signaltechnischen und/oder energietechnischen Kopplung zweier Bauteile kann vorzugsweise verstanden werden, dass die entsprechende Elektroniken dieser Bauteile miteinander elektrisch verbunden sind und vorzugsweise ausgebildet sind, um Signale und/oder Energie zu übertragen. Eine Übertragung von Signalen und/oder Energie kann vorzugsweise als Senden und/oder Empfangen von Signalen und/oder Energie verstanden werden.

Das Motorkopfstück, die Adapterstücke und die Kommunikatorstücke sind jeweils separate Bauteile, die miteinander gekoppelt werden können. Vorzugsweise kann jedes Kommunikatorstück mit den Adapterstücken kombiniert werden.

Durch unterschiedliche Adapterstücke, die entsprechend der Feldgeräte konfiguriert sind, kann die Datenübertragungseinheit individuell an Kundenbedürfnisse angepasst werden.

Durch die vorgeschlagene Lösung besteht keine Notwendigkeit, dass die Auswerteeinheit für jedes Feldgerät eine eigene Auswerteelektronik umfasst. Durch das Senden, vorzugsweise standardisierter, Signale, die Messinformationen und Feldgerätinformationen tragen, können insbesondere die Signale die über die verschiedenen Kommunikationseinheiten und das Motorkopfstück gesendet werden vorzugsweise mit einer einzigen Auswerteelektronik ausgewertet werden.

Durch die vorgeschlagene Lösung mit angepassten Adapterstücken muss auf Hardwareseite nur ein entsprechend konfiguriertes Adapterstück entworfen werden - der hardwareseitige Aufwand ein neues Feldgerät in ein System zu integrieren ist also deutlich geringer.

Durch die Nutzung des Adapterstücks in unmittelbarer Nähe des Feldgeräts und insbesondere durch die Umsetzung Signale direkt am Ort der Entstehung in unanfälligere analoge und/oder digitale Signale, kann die Verarbeitung der Signale robuster gestaltet werden.

Vorteilhaft an dieser Lösung ist ferner, dass durch das Adapterstück eine Art Standardisierung erzielt werden kann, die eine Vereinfachung der Verarbeitung der Signale zur Folge hat. Darüber hinaus können Komponenten, die für eine Aufbereitung der Signale benötigt werden, reduziert werden.

Vorzugsweise kann der Motorkopf mit einem Zuleitungskabel elektrisch verbindbar oder verbunden sein. Dieses Zuleitungskabel kann vorzugsweise, neben evtl. vorhandenen Adern zur Energieversorgung der Antriebseinheit, vier Adern, zwei zur Energieversorgung, insbesondere der Motorkopfelektronik und zwei zur Datenübertragung, umfassen. Die Adern zur Datenübertragung können beispielsweise dem RS485-Standard entsprechen. Dadurch kann eine besonders robuste und für Fehler unanfällige Kommunikation ermöglicht werden.

Die Motorkopfelektronik kann vorzugsweise ausgebildet sein, um Signale über beispielsweise Modbus zur Auswerteeinheit zu senden oder von dieser zu empfangen. Die Motorkopfelektronik kann ferner ausgebildet sein, um im Bridge-Betrieb zwischen den Feldgeräten und der Auswerteeinheit bzw. einer Steuereinheit und zusätzlich als Hub für Feldgeräte an einem Bioreaktor zu arbeiten.

Insbesondere bevorzugt kann die Datenübertragungseinheit die Auswerteeinheit umfassen, wobei die Auswerteeinheit mit dem Motorkopfstück signaltechnisch gekoppelt ist.

Die Auswerteeinheit kann vorzugsweise ausgebildet sein, um Signale, vorzugsweise von der Datenübertragungseinheit, zu empfangen und diese auszuwerten. Ferner kann die Auswerteeinheit ausgebildet sein, um Signale zu erzeugen und an die Datenübertragungseinheit zu senden. Vorzugsweise können diese Signale dazu dienen, die Feldgeräte anzusteuern und/oder zu kalibrieren. Insbesondere bevorzugt, kann die Auswerteeinheit Signale von der Datenübertragungseinheit empfangen, diese Signale auswerten und in Abhängigkeit dieser Signale Signale erzeugen und an die Datenübertragungseinheit senden.

Vorzugsweise kann die Auswerteeinheit eine Bedienerschnittstelle aufweisen, die ein Bedienen der Datenübertragungseinheit durch ein Bedienpersonal ermöglicht. Dadurch können vorzugsweise manuell Feldgeräte angesteuert und/oder Daten ausgelesen werden. Ferner kann dadurch vorzugsweise die Antriebseinheit durch das Bedienpersonal angesteuert werden.

Vorzugsweise kann die Datenübertragungseinheit für eine Kopfplatte eines Bioreaktors das Motorkopfstück, das mit einer Auswerteeinheit signaltechnisch und mit einer Antriebseinheit, die an einer Kopfplatte eines Bioreaktors anordenbar ist, energietechnisch koppelbar ist, umfassend eine Motorkopfelektronik, und eine einzige mit dem Motorkopfstück signaltechnisch gekoppelte Kommunikationseinheit aufweisen, umfassend: ein Adapterstück mit einer Adapterelektronik, die mit einer Feldgerätelektronik eines Feldgeräts elektrisch verbindbar ist, und ein Kommunikatorstück mit einer Kommunikatorelektronik, die mit der Adapterelektronik und der Motorkopfelektronik elektrisch verbunden ist, wobei die Adapterelektronik einen Signalwandler umfasst und eingerichtet ist, um Signale zwischen der Feldgerätelektronik und der Kommunikatorelektronik zu kommunizieren, wobei die Signale Feldgerätinformationen und Messinformationen und/oder Steuerinformation tragen, wobei die Kommunikatorelektronik eingerichtet ist, um die Signale zwischen der Adapterelektronik und der Motorkopfelektronik zu kommunizieren, wobei das Adapterstück und das Kommunikatorstück als separate, miteinander koppelbare Bauteile ausgebildet sind, wobei das Motorkopfstück als ein separates, mit der Kommunikationseinheit koppelbares Bauteil ausgebildet ist.

Gemäß einer bevorzugten Fortbildung der Datenübertragungseinheit umfasst das Adapterstück eine Feldgerätschnittstelle und eine Kommunikatorschnittstelle, wobei die Adapterelektronik zwischen die Feldgerätschnittstelle und die Kommunikatorschnittstelle geschaltet ist, über die Feldgerätschnittstelle mit der Feldgerätelektronik elektrisch verbindbar ist und über die Kommunikatorschnittstelle mit der Kommunikatorelektronik elektrisch verbunden ist, und das Kommunikatorstück eine Adapterschnittstelle und eine Motorkopfschnittstelle umfasst, wobei die Kommunikatorelektronik zwischen die Adapterschnittstelle und die Motorkopfschnittstelle geschaltet ist, über die Adapterschnittstelle mit der Adapterelektronik elektrisch verbunden ist und über die Motorkopfschnittstelle mit der Motorkopfelektronik elektrisch verbunden ist.

Die Adapterelektronik kann vorzugsweise ausgebildet sein, um Signale über die Feldgerätschnittstelle und/oder die Kommunikatorschnittstelle zu senden und/oder zu empfangen. Hierbei kann die Adapterelektronik insbesondere ausgebildet sein, um Signale der Feldgerätschnittstelle in Signale der Kommunikatorschnittstelle umzusetzen. Ferner kann die Adapterelektronik insbesondere ausgebildet sein, um Signale der Kommunikatorschnittstelle in Signale der Feldgerätschnittstelle umzusetzen.

Die Kommunikatorelektronik kann vorzugsweise ausgebildet sein, um Signale über die Adapterschnittstelle und/oder die Motorkopfschnittstelle zu senden und/oder zu empfangen. Hierbei kann die Kommunikatorelektronik insbesondere ausgebildet sein, um Signale der Adapterschnittstelle in Signale der Motorkopfschnittstelle umzusetzen. Ferner kann die Kommunikatorelektronik insbesondere ausgebildet sein, um Signale der Motorkopfschnittstelle in Signale der Adapterschnittstelle umzusetzen.

Bei der Feldgerätschnittstelle und/oder der Kommunikatorschnittstelle und/oder der Adapterschnittstelle und/oder der Motorkopfschnittstelle kann es sich vorzugsweise um Hardwareschnittstellen handeln, die ausgebildet sind, um physische Systeme in der Elektrotechnik und Elektronik miteinander zu verbinden. Hierdurch können vorzugsweise die einzelnen Bauteile, insbesondere die Elektroniken der einzelnen Bauteile elektrisch, miteinander verbunden werden.

Die Feldgerätschnittstelle und/oder die Kommunikatorschnittstelle und/oder die Adapterschnittstelle und/oder die Motorkopfschnittstelle können vorzugsweise als analoge und/oder digitale Schnittstellen ausgebildet sein. Insbesondere können die Feldgerätschnittstelle und/oder die Kommunikatorschnittstelle und/oder die Adapterschnittstelle und/oder die Motorkopfschnittstelle als USB, FireWire, ElA-232, PCI-Express, SPI, I2C, RS232, RS485, DMX, Serial ATA oder Profibus ausgebildet sein. Besonders bevorzugt können die Feldgerätschnittstelle und/oder die Kommunikatorschnittstelle und/oder die Adapterschnittstelle und/oder die Motorkopfschnittstelle als RS485 Schnittstelle ausgebildet sein.

Vorzugsweise kann die Adapterelektronik auf einer Leiterplatte untergebracht sein. Die Leiterplatte kann vorzugsweise Pins aufweisen. Die Leiterplatte kann vorzugsweise mindestens zwei Pins aufweisen, die konfiguriert sind, um eine elektrische Verbindung zwischen der Adapterelektronik und der Feldgerätelektronik zu ermöglichen, wobei diese Pins vorzugsweise als Ausgänge und/oder Eingänge eingerichtet sind. Darüber hinaus kann die Leiterplatte vorzugsweise zwei Pins aufweisen, die zur Energieversorgung eingerichtet sind. Die Leiterplatte kann insbesondere zwei Federkontakte aufweisen, die eine elektrische Verbindung zwischen der Adapterelektronik und der Kommunikatorelektronik ermöglichen.

Weitere konfigurierte Pins können auf der Leiterplatte der Adapterelektronik fest kodiert sein und entsprechende Bitmuster an die Kommunikatorelektronik weitergeben. Alternativ oder zusätzlich kann diese Weitergabe auch über ein entsprechendes Protokoll erfolgen wobei dabei dann vorzugsweise bereits digitale Signale in der Adapterelektronik vorliegen.

Ferner vorzugsweise kann die Kommunikatorelektronik auf einer Leiterplatte und/oder die Motorkopfelektronik auf einer Leiterplatte untergebracht sein.

Insbesondere bevorzugt umfasst die Datenübertragungseinheit mindestens eine erste Gruppe von ersten Adapterstücken und eine zweite Gruppe von zweiten Adapterstücken, wobei die Adapterelektronik der ersten Adapterstücke ausgebildet ist, um die Signale zwischen der Feldgerätelektronik eines ersten Feldgeräts und der Kommunikatorelektronik zu kommunizieren, wobei die Adapterelektronik der zweiten Adapterstücke ausgebildet ist, um die Signale zwischen der Feldgerätelektronik eines zweiten Feldgeräts und der Kommunikatorelektronik zu kommunizieren, wobei das Adapterstück jeder Kommunikationseinheit in Abhängigkeit eines zu koppelnden Feldgeräts ausgewählt ist aus mindestens der ersten Gruppe von ersten Adapterstücken und der zweiten Gruppe von zweiten Adapterstücken.

Gemäß dieser bevorzugten Ausführungsform wird ein modulares System bereitgestellt. Dieses System umfasst vorzugweise unterschiedliche Gruppen von Adapterstücken, die vorzugsweise entsprechend unterschiedlicher Feldgeräte konfiguriert sind. Die Adapterstücke können insbesondere in Abhängigkeit eines Feldgeräts ausgewählt und mit diesem gekoppelt werden. Dadurch können unterschiedliche Feldgeräte eingesetzt werden.

Die Kommunikatorstücke können vorzugsweise im Wesentlichen identisch sein und ausgebildet sein, um mit jedem der Adapterstücke gekoppelt zu werden.

Insbesondere kann somit eine Datenübertragungseinheit bereitgestellt werden, die individuell an Kundenbedürfnisse angepasst ist. Vorteilhaft ist ferner, dass eine Datenverarbeitungseinheit auch umgerüstet und/oder aufgerüstet werden kann.

Gemäß einer weiter bevorzugten Ausführungsvariante umfasst das Adapterstück eine Feldgerätkupplung, die ausgebildet ist, um das Feldgerät mit dem Adapterstück mechanisch, vorzugsweise lösbar, zu verbinden. Insbesondere bevorzugt kann das Adapterstück auf das Feldgerät aufgeschraubt werden.

Die Feldgerätkupplung kann vorzugsweise an der Feldgerätschnittstelle ausgebildet sein. Insbesondere bevorzugt kann dadurch das Feldgerät mit dem Adapterstück sowohl elektrisch als auch mechanisch verbunden werden.

Dadurch kann das Adapterstück besonders einfach an das Feldgerät angeschlossen werden. Ferner ermöglicht die mechanische Verbindung dieser Komponenten eine besonders kompakte Feldgerät-Adapterstück-Einheit, die eine einfache Handhabung ermöglicht.

Die lösbare Verbindung des Adapterstücks mit dem Feldgerät kann vorzugsweise ein Austauschen des Feldgeräts und/oder des Adapterstücks ermöglichen.

Ferner ist es bevorzugt, dass das Kommunikatorstück eine Adapterkupplung umfasst, die ausgebildet ist, um das Adapterstück mit dem Kommunikatorstück mechanisch, vorzugsweise lösbar, zu verbinden.

Die Adapterkupplung kann vorzugsweise an der Adapterschnittstelle ausgebildet sein. Insbesondere bevorzugt kann dadurch das Kommunikatorstück mit dem Adapterstück sowohl elektrisch als auch mechanisch verbunden werden.

Dadurch kann das Kommunikatorstück besonders einfach an das Adapterstück angeschlossen werden. Ferner ermöglicht die mechanische Verbindung dieser Komponenten eine besonders kompakte Kommunikatorstück-Adapterstück-Einheit, die eine einfache Handhabung ermöglicht.

Die lösbare Verbindung des Kommunikatorstücks mit dem Adapterstück kann vorzugsweise ein Austauschen des Kommunikatorstücks und/oder des Adapterstücks ermöglichen.

Insbesondere ist es bevorzugt, dass das Adapterstück eine Feldgerätkupplung umfasst, die ausgebildet ist, um das Feldgerät mit dem Adapterstück mechanisch, vorzugsweise lösbar, zu verbinden, und/oder das das Kommunikatorstück eine Adapterkupplung umfasst, die ausgebildet ist, um das Adapterstück mit dem Kommunikatorstück mechanisch, vorzugsweise lösbar, zu verbinden.

Insbesondere bevorzugt kann die Feldgerätkupplung ausgebildet sein, um das Feldgerät mit dem Adapterstück mittels einer Bajonett-, Schraub- oder Clickverbindung zu verbinden. Vorzugsweise kann die Feldgerätkupplung an einem Adapterstück-Gehäuse ausgebildet sein. Insbesondere kann das Feldgerät eine Adapterkupplung aufweisen, die ausgebildet ist, um mit der Feldgerätkupplung in Eingriff gebracht zu werden.

Insbesondere bevorzugt kann die Adapterkupplung ausgebildet sein, um das Kommunikatorstück mit dem Adapterstück mittels einer Bajonett-, Schraub- oder Clickverbindung zu verbinden. Vorzugsweise kann die Adapterkupplung an einem Kommunikatorstück-Gehäuse ausgebildet sein. Insbesondere kann das Adapterstück eine Kommunikatorkupplung aufweisen, die ausgebildet ist, um mit der Adapterkupplung in Eingriff gebracht zu werden.

Gemäß einer weiter bevorzugten Ausführungsvariante ist der Signalwandler ein Analog-Digital-Umsetzer. Ferner ist vorzugsweise die Adapterelektronik und/oder die Kommunikatorelektronik und/oder der Signalwandler eingerichtet, um analoge Signale zu empfangen, in normierte analoge Signale umzuwandeln und die normierten analogen Signale in digitale Signale umzusetzen und die digitalen Signale zu senden zu senden und/oder umgekehrt.

Durch diese Ausführungsvariante können analoge Signale, die vorzugsweise von der Feldgerätelektronik erzeugt und an die Adapterelektronik gesendet werden, in der Adapterelektronik und/oder der Kommunikatorelektronik in digitale Signale umgesetzt werden. Digitale Signale sind in der Regel weniger anfällig für Störungen bei einer Übertragung, da Signalpegel der digitalen Signale mit einer gewissen Toleranz immer noch einem korrekten Wert zugeordnet werden können. Insbesondere differentielle digitale Signale sind hier besonders robust gegen äußere Störungen.

Vorzugsweise kann die Kommunikatorelektronik einen Repeater und/oder Signalaufbereiter umfassen und ausgebildet sein, um digitale Signale zu empfangen, die digitalen Signale mittels des Repeaters und/oder des Signalaufbereiters aufzubereiten und die aufbereiteten digitalen Signale zu senden. Dadurch können gegebenenfalls Fehler korrigiert, insbesondere unerwünschtes Rauschen entfernt, werden. Vorteilhaft daran ist, dass durch die Aufbereitung der digitalen Signale in der Kommunikatorelektronik vorzugsweise nur das Nutzsignal weiter an die Motorkopfelektronik gesendet werden kann.

Insbesondere kann auch die Motorkopfelektronik einen Repeater und/oder Signalaufbereiter umfassen, um die von den Kommunikatorstücken empfangenen digitalen Signale aufzubereiten.

Durch die Umsetzung analoger Signale in digitale Signale kann eine robustere Signalübertragung ermöglicht werden.

Vorzugsweise kann der Signalwandler auch einen Repeater und/oder Signalaufbereiter umfassen, der eingerichtet ist, um digitale Signale aufzubereiten. Diese Ausführungsvariante ist insbesondere dann vorteilhaft, wenn das Adapterstück eingerichtet ist, um digitale Signale von dem Feldgerät zu empfangen und/oder digitale Signale von dem Kommunikatorstück zu empfangen und an das Feldgerät zu senden.

Vorzugsweise können zur analogen Übertragung Stromsignale, insbesondere 0 mA - 20 mA oder mit versetzten Nullpunkt 4 mA - 20 mA, oder Spannungssignale, insbesondere < 0 V - 5 V, 0 V - 10 V, mit versetzten Nullpunkt 1 V - 5 V, mit versetzten Nullpunkt 2 V - 10 V oder mit oder ohne versetzten Nullpunkt -10 V - 10 V, verwendet werden.

Insbesondere bevorzugt können die Stromsignale als normierte Signale verwendet werden. Diese sind besonders unempfindlich gegenüber elektromagnetischen Störungen und Spannungsverlusten.

Insbesondere können analoge Sensorsignale des Feldgeräts in normierte Signale (zum Beispiel 0 V bis 4,095 V) umwandelt werden und als umgesetzte digitale Signale (zum Beispiel 0 -4095) weitergesendet werden.

Ferner ist es bevorzugt, wenn die Kommunikatorelektronik und/oder die Motorkopfelektronik dazu eingerichtet ist, die Signale kontaktlos oder kontaktgebunden, vorzugsweise kabelgebunden, zu empfangen, die Signale in Funksignale umzusetzen und per Funk oder kabelgebunden zu senden und/oder umgekehrt. Dadurch kann die die Signalübertragung zwischen der Kommunikatorelektronik und der Motorkopfelektronik kabellos erfolgen. Durch diese Ausgestaltung kann eine Kabelage an der Datenübertragungseinheit reduziert oder vermieden werden. Dadurch wird auch die Handhabbarkeit und insbesondere das Anbringen der Datenübertragungseinheit insbesondere an die Kopfplatte des Bioreaktors besonders vereinfacht.

Gemäß dieser Ausführungsvariante kann das Kommunikatorstück und/oder das Motorkopfstück eine Antenne aufweisen, die eingerichtet ist, um die Funksignale zu senden und/oder zu empfangen. Die Antenne kann vorzugsweise biegbar sein. Dadurch kann ein möglichst guter Schluss der Felder zwischen dem Motorkopfstück und dem Kommunikatorstück erzielt werden.

Das Umsetzen in Funksignale kann beispielsweise mittels Modulation erfolgen. Insbesondere kann durch Demodulation das ursprüngliche Signal bzw. die Information des ursprünglichen Signals wiedergewonnen und gegebenenfalls weitergeleitet und/oder weiterverarbeitet werden.

Durch diese Ausführungsvariante müssen keine elektrischen Kabel zwischen dem Kommunikatorstück und dem Motorkopfstück verlegt werden.

Weiter ist es bevorzugt, wenn die Adapterelektronik dazu eingerichtet ist, die Signale kontaktlos oder kontaktgebunden, vorzugsweise kabelgebunden, zu empfangen, die Signale in Funksignale umzusetzen und per Funk oder kabelgebunden zu senden und/oder umgekehrt. Dadurch kann die die Signalübertragung zwischen der Adapterelektronik und der Kommunikatorelektronik kabellos erfolgen. Durch diese Ausgestaltung kann eine Kabelage an der Datenübertragungseinheit reduziert oder vermieden werden. Dadurch wird auch die Handhabbarkeit und insbesondere das Anbringen der Datenübertragungseinheit insbesondere an die Kopfplatte des Bioreaktors besonders vereinfacht.

Gemäß dieser Ausführungsvariante kann das Kommunikatorstück und/oder das Adapterstück eine Antenne aufweisen, die eingerichtet ist, um die Funksignale zu senden und/oder zu empfangen. Die Antenne kann vorzugsweise biegbar sein. Dadurch kann ein möglichst guter Schluss der Felder zwischen dem Adapterstück und dem Kommunikatorstück erzielt werden. Eine weitere mögliche Ausprägung ist eine integrierte Antenne auf der Leiterplatte von der Kommunikatorelektronik und/oder auf der Leiterplatte von der Adapterelektronik.

Das Umsetzen in Funksignale kann beispielsweise mittels Modulation erfolgen. Insbesondere kann durch Demodulation das ursprüngliche Signal bzw. die Information des ursprünglichen Signals wiedergewonnen und gegebenenfalls weitergeleitet und/oder weiterverarbeitet werden.

Durch diese Ausführungsvariante müssen keine elektrischen Kabel zwischen dem Kommunikatorstück und dem Adapterstück verlegt werden.

Zum Umsetzen der Signale, vorzugsweise der digitalen Signale, in Funksignale und/oder umgekehrt, kann ein Modem eingesetzt werden. Vorzugsweise kann die Kommunikatorelektronik und/oder die Adapterelektronik das Modem aufweisen, das eingerichtet ist, um digitale Signale in Funksignale umzusetzen und/oder umgekehrt.

Ferner vorzugsweise kann die Adapterelektronik und/oder die Kommunikatorelektronik ausgebildet sein, um Funksignale zu empfangen, in ursprüngliche, digitale Signale umzusetzen und die ursprünglichen, digitalen Signale zu übertragen.

Vorzugsweise können die Signale zwischen der Adapterelektronik und der Kommunikatorelektronik kontaktgebunden, vorzugsweise kabelgebunden, oder über Funk übertragen werden und/oder umgekehrt. Insbesondere bevorzugt können die Signale zwischen der Kommunikatorelektronik und der Motorkopfelektronik kontaktgebunden, vorzugsweise kabelgebunden, oder über Funk übertragen werden und/oder umgekehrt.

Allgemein können Funksignale vorzugsweise auf einem Bluetooth-Protokoll oder davon abgewandelten Protokoll, insbesondere Bluetooth Low Energy, ZigBee, 6LoWPAN und/oder einem (anderen) Wireless-Protokoll basieren. Insbesondere kann eine Funkverbindung mit sehr kurzer Reichweite und somit einer geringen Sendeleistung erfolgen.

Allgemein kann eine kabelgebundene Verbindung vorzugsweise durch ein sehr kurzes, insbesondere halbflexibles, Kabel realisiert werden. Insbesondere kann ein Kabel, das am Kommunikator mittels eines zu fixierenden, vorzugsweise schraubbar zu fixierenden, Steckers befestigt wird, eingesetzt werden. Beispielsweise könnte eine Gegenseite des Kabels mit dem Motorkopfstück oder am Adapterstück gekoppelt werden, vorzugsweise mittels eines, beispielsweise keilförmig zulaufenden, Steckers, und/oder in Form eines Federkontakts. Vorzugsweise erfolgt eine lösbare Sicherung der Verbindung zwischen Kabel und Motorkopf, beispielsweise durch eine Rastverbindung und/oder eine Magnetverbindung. Hierdurch kann zum einen eine schnelle und einfache Kopplung und zum anderen ein manuelles Lösen einer durch den Einrastmechanismus erzielten Sperre gewährleistet werden.

Gemäß einer besonders bevorzugten Ausführungsvariante kann die Datenübertragungseinheit das Motorkopfstück, das mit der Auswerteeinheit signaltechnisch und mit der Antriebseinheit, die an der Kopfplatte des Bioreaktors anordenbar ist, energietechnisch koppelbar ist, umfassend die Motorkopfelektronik, und die mindestens zwei, jeweils mit dem Motorkopfstück signaltechnisch gekoppelten Kommunikationseinheiten aufweisen, jeweils umfassend: das Adapterstück mit der Adapterelektronik, die mit der Feldgerätelektronik des Feldgeräts elektrisch verbindbar ist, und das Kommunikatorstück mit der Kommunikatorelektronik, die mit der Adapterelektronik und der Motorkopfelektronik elektrisch verbunden ist, wobei die Adapterelektronik den Analog-Digital-Umsetzer umfasst und eingerichtet ist, um analoge Signale zu empfangen, in digitale Signale umzusetzen und die digitalen Signale zu senden und/oder umgekehrt, um die Signale zwischen der Feldgerätelektronik und der Kommunikatorelektronik zu kommunizieren, wobei die Signale Feldgerätinformationen und Messinformationen und/oder Steuerinformation tragen, wobei die Kommunikatorelektronik eingerichtet ist, um die Signale zwischen der Adapterelektronik und der Motorkopfelektronik zu kommunizieren, wobei das Adapterstück und das Kommunikatorstück als separate, miteinander koppelbare Bauteile ausgebildet sind, wobei das Motorkopfstück als ein separates, mit den Kommunikationseinheiten koppelbares Bauteil ausgebildet ist.

Durch diese Ausgestaltung können Feldgeräte eingesetzt werden, die analoge und insbesondere besonders anfällige Signale erzeugen und diese an das Adapterstück senden. Dadurch können die Adapterstücke an herkömmliche Feldgeräte, die keine digitalen Signale erzeugen können, angeschlossen werden.

Durch die Umsetzung dieses Signals am Ort der Entstehung, kann eine, insbesondere robustere, Signalübertragung ermöglicht werden. Die digitalen Signale sind klarer als analoge Signale und genau definiert. Somit sind diese auch leichter rekonstruierbar, haben eine hohe Störsicherheit und ermöglichen eine flexible Weiterverarbeitung.

Gemäß einer alternativen besonders bevorzugten Ausführungsvariante kann die Datenübertragungseinheit das Motorkopfstück, das mit der Auswerteeinheit signaltechnisch und mit der Antriebseinheit, die an der Kopfplatte des Bioreaktors anordenbar ist, energietechnisch koppelbar ist, umfassend die Motorkopfelektronik, und die mindestens zwei, jeweils mit dem Motorkopfstück signaltechnisch gekoppelten Kommunikationseinheiten aufweisen, jeweils umfassend: das Adapterstück mit der Adapterelektronik, die mit der Feldgerätelektronik des Feldgeräts elektrisch verbindbar ist, und das Kommunikatorstück mit der Kommunikatorelektronik, die mit der Adapterelektronik und der Motorkopfelektronik elektrisch verbunden ist, wobei die Adapterelektronik den Analog-Digital-Umsetzer umfasst und eingerichtet ist, um analoge Signale zu empfangen, in normierte Signale umzusetzen und die normierten Signale zu senden und/oder umgekehrt, um die Signale zwischen der Feldgerätelektronik und der Kommunikatorelektronik zu kommunizieren, wobei die Signale Feldgerätinformationen und Messinformationen und/oder Steuerinformation tragen, wobei die Kommunikatorelektronik eingerichtet ist, um die Signale zwischen der Adapterelektronik und der Motorkopfelektronik zu kommunizieren, wobei das Adapterstück und das Kommunikatorstück als separate, miteinander koppelbare Bauteile ausgebildet sind, wobei das Motorkopfstück als ein separates, mit den Kommunikationseinheiten koppelbares Bauteil ausgebildet ist.

Durch diese Ausgestaltung können Feldgeräte eingesetzt werden, die analoge und insbesondere besonders anfällige Signale erzeugen und diese an das Adapterstück senden. Dadurch können die Adapterstücke an herkömmliche Feldgeräte, die keine digitalen Signale erzeugen können, angeschlossen werden.

Durch die Umsetzung der Signale des Feldgeräts in Einheitssignale können diese weitergeleitet und verarbeitet werden. Darüber hinaus können aufgrund der Normierung standardisierte Kommunikatorstücke eingesetzt werden, die mit unterschiedlichen Adapterstücken kommunizieren können.

Insbesondere bevorzugt können Stromsignale als normierte Signale verwendet werden. Diese sind besonders unempfindlich gegenüber elektromagnetischen Störungen und Spannungsverlusten. Dadurch kann eine robustere Signalübertragung ermöglicht werden. Ferner kann dadurch der Analog-Digital-Umsetzer selbst permanent mit Energie versorgt werden.

Gemäß einer alternativen besonders bevorzugten Ausführungsvariante kann die Datenübertragungseinheit das Motorkopfstück, das mit der Auswerteeinheit signaltechnisch und mit der Antriebseinheit, die an der Kopfplatte des Bioreaktors anordenbar ist, energietechnisch koppelbar ist, umfassend die Motorkopfelektronik, und die mindestens zwei, jeweils mit dem Motorkopfstück signaltechnisch gekoppelten Kommunikationseinheiten aufweisen, jeweils umfassend: das Adapterstück mit der Adapterelektronik, die mit der Feldgerätelektronik des Feldgeräts elektrisch verbindbar ist, und das Kommunikatorstück mit der Kommunikatorelektronik, die mit der Adapterelektronik und der Motorkopfelektronik elektrisch verbunden ist, wobei die Adapterelektronik den Signalwandler, vorzugsweise den Analog-Digital-Umsetzer, umfasst und eingerichtet ist, um Signale zwischen der Feldgerätelektronik und der Kommunikatorelektronik zu kommunizieren, vorzugsweise um analoge Signale zu empfangen, in normierte Signale umzusetzen und die normierten Signale zu senden und/oder umgekehrt, wobei die Signale Feldgerätinformationen und Messinformationen und/oder Steuerinformation tragen, wobei die Kommunikatorelektronik den Analog-Digital-Wandler umfasst und eingerichtet ist, um analoge Signale zu empfangen, in digitale Signale umzusetzen und die digitalen Signale zu senden und/oder umgekehrt, um die Signale zwischen der Adapterelektronik und der Motorkopfelektronik zu kommunizieren, wobei das Adapterstück und das Kommunikatorstück als separate, miteinander koppelbare Bauteile ausgebildet sind, wobei das Motorkopfstück als ein separates, mit den Kommunikationseinheiten koppelbares Bauteil ausgebildet ist.

Jede der vorstehend besonders bevorzugten Ausführungsvarianten gewährleistet eine robustere Signalübertragung. Einer robusten Signalübertragung und damit einhergehend exakten Übertragung der Informationen kommt in der Bioprozesstechnik eine herausragende Bedeutung zu. Bei der Kultivierung von Mikroorganismen und/oder Zellkulturen kommt es maßgeblich auf jeden einzelnen Parameter an. So ist beispielsweise die Zusammensetzung des Kulturmediums, dessen pH-Wert und Temperatur von besonderer Bedeutung. So können bereits geringe Schwankungen Auswirkungen auf die Wachstumskurve haben. Darüber hinaus ist zu bedenken, dass die Mikroorganismen und/oder Zellkulturen Nährstoffe des Kulturmediums verbrauchen. Daher besteht die Notwendigkeit die Nährstoffkonzentrationen, vorzugsweise in Echtzeit, exakt zu überwachen, um eine Nährstoffzufuhr regeln zu können. Ferner können Mikroorganismen und/oder Zellkulturen durch den Stoffwechsel eine unterschiedliche Schaumbildung zur Folge haben. Insbesondere um eine zu starke Schaumbildung zu vermeiden, die auch ein Überquellen des Bioreaktors zur Folge haben kann, muss die Schaumbildung in Echtzeit überwacht werden, um diese durch Einstellen anderer Parameter zu kontrollieren.

Gemäß einer alternativen besonders bevorzugten Ausführungsvariante kann die Datenübertragungseinheit das Motorkopfstück, das mit der Auswerteeinheit signaltechnisch und mit der Antriebseinheit, die an der Kopfplatte des Bioreaktors anordenbar ist, energietechnisch koppelbar ist, umfassend die Motorkopfelektronik, und die mindestens zwei, jeweils mit dem Motorkopfstück signaltechnisch gekoppelten Kommunikationseinheiten aufweisen, jeweils umfassend: das Adapterstück mit der Adapterelektronik, die mit der Feldgerätelektronik des Feldgeräts elektrisch verbindbar ist, und das Kommunikatorstück mit der Kommunikatorelektronik, die mit der Adapterelektronik und der Motorkopfelektronik elektrisch verbunden ist, wobei die Adapterelektronik den Signalwandler umfasst und eingerichtet ist, um die Signale zwischen der Feldgerätelektronik und der Kommunikatorelektronik zu kommunizieren, wobei die Signale Feldgerätinformationen und Messinformationen und/oder Steuerinformation tragen, wobei die Kommunikatorelektronik eingerichtet ist, um die Signale kontaktgebunden, vorzugsweise kabelgebunden, zu empfangen, die Signale in Funksignale umzusetzen und per Funk zu senden und/oder umgekehrt, um die Signale zwischen der Adapterelektronik und der Motorkopfelektronik zu kommunizieren, wobei das Adapterstück und das Kommunikatorstück als separate, miteinander koppelbare Bauteile ausgebildet sind, wobei das Motorkopfstück als ein separates, mit den Kommunikationseinheiten koppelbares Bauteil ausgebildet ist.

Gemäß dieser bevorzugten Ausgestaltung kann der Signalwandler vorzugsweise ein Analog-Digital-Umsetzer oder ein Repeater und/oder Signalaufbereiter sein. Insbesondere für den Fall, dass das Adapterstück an einen analogen Sensor anschließbar ist, kann der Signalwandler als Analog-Digital-Wandler ausgebildet sein. Insbesondere für den Fall, dass das Adapterstück an einen digitalen Sensor anschließbar ist, kann der Signalwandler als Repeater und/oder Signalaufbereiter ausgebildet sein.

Alternativ kann beispielsweise der Signalwandler ein Analog-Digital-Umsetzer sein. Hierbei kann die Kommunikatorelektronik einen Analog-Digital-Wandler umfassen und eingerichtet sein, um normierte analoge Signale zu empfangen, in digitale Signale umzusetzen und die digitalen Signale in Funksignale umzuwandeln.

Diese bevorzugte Ausführungsvariante verbindet die zuvor ausgeführten Vorteile hinsichtlich der unterschiedlichen Ausgestaltung der Signalwandler und der Vorteile der Funkübertragung. Insbesondere kann dadurch eine Kabelage an der Datenübertragungseinheit reduziert oder vermieden werden. Dadurch wird auch die Handhabbarkeit und insbesondere das Anbringen der Datenübertragungseinheit, insbesondere an die Kopfplatte des Bioreaktors besonders vereinfacht.

Gemäß einer weiter bevorzugten Ausführungsvariante ist vorgesehen, dass die Feldgerätschnittstelle und/oder die Kommunikatorschnittstelle und/oder die Adapterschnittstelle und/oder die Motorkopfschnittstelle als ein Anschluss an einem Kabel ausgebildet ist, wobei vorzugsweise das Kabel eine Länge von maximal 20 cm aufweist.

Der Anschluss kann insbesondere als ein an einem Kabel angeordneten Stecker ausgebildet sein.

Das Kabel kann vorzugsweise eine maximale Länge von 50 cm, 20 cm, 15 cm, 10 cm, 8 cm, 6 cm, 5 cm oder 4 cm aufweisen. Vorzugsweise ist die maximale Länge des Kabels abhängig von den Dimensionen des Bioreaktors.

Durch die Kabel mit einer besonders kurzen Länge kann zum einen die Handhabbarkeit verbessert werden, da keine unnötigen langen Kabel zum Einsatz kommen und daher eine Kabelage reduziert wird. Dies kann vorzugsweise auch zu einer ordentlichen äußeren Erscheinung führen. Darüber hinaus kann die Signalübertragung verbessert werden.

Insbesondere bevorzugt ist, dass das Motorkopfstück mit dem Kommunikatorstück energietechnisch gekoppelt ist, wobei die Motorkopfelektronik eingerichtet ist, um die Kommunikatorelektronik mit Energie, insbesondere Strom, zu versorgen, und/oder das Kommunikatorstück mit dem Adapterstück energietechnisch gekoppelt ist, wobei die Kommunikatorelektronik eingerichtet ist, um die Adapterelektronik mit Energie, insbesondere Strom, zu versorgen, und/oder das Adapterstück mit dem Feldgerät energietechnisch koppelbar ist, wobei die Adapterelektronik eingerichtet ist, um die Feldgerätelektronik mit Energie, insbesondere Strom, zu versorgen, wobei vorzugsweise der Strom kontaktgebunden, vorzugsweise kabelgebunden, und/oder induktiv übertragen wird.

Die bevorzugte induktive Energieversorgung kann vorzugsweise von einer induktiven Quelle im Motorkopfstück gespeist sein.

Vorzugsweise kann die Motorkopfelektronik und/oder die Kommunikatorelektronik und/oder die Adapterelektronik eine Versorgungselektronik umfassen. Das Motorkopfstück kann insbesondere als ein Stromverteiler eingerichtet sein, um sämtliche Kommunikatorelektroniken, die mit der Motorkopfelektronik elektrisch verbunden sind mit Strom zu versorgen. Dadurch kann ein besonders einfacher Aufbau der Datenübertragungseinheit gewährleistet werden. Insbesondere können die einzelnen Komponenten, also vorzugsweise das Adapterstück und/oder das Kommunikatorstück, besonders einfach ausgestaltet werden. Dadurch entfällt auch die Notwenigkeit die einzelnen Komponenten mit einer Versorgungsleitung elektrisch zu verbinden oder andere Energiequellen in den Komponenten vorzusehen.

Durch diese Ausgestaltung können beispielsweise auch die Feldgeräte mit Energie versorgt werden.

Gemäß einer weiter bevorzugten Ausführungsvariante umfasst das Motorkopfstück eine Kommunikationsschnittstelle, die mit der Kommunikatorelektronik elektrisch verbunden ist, und eine Zuleitungsschnittstelle, an die ein Zuleitungskabel elektrisch anschließbar ist, wobei die Zuleitungsschnittstelle mit einer Auswerteelektronik der Auswerteeinheit elektrisch verbindbar ist, wobei die Motorkopfelektronik zwischen die Kommunikationsschnittstelle und die Zuleitungsschnittstelle geschaltet ist, wobei die Motorkopfelektronik eingerichtet ist, um Signale zwischen der mit der Kommunikationsschnittstelle elektrisch verbundenen Kommunikatorelektronik und einer mit der Zuleitungsschnittstelle elektrisch verbindbaren Auswerteelektronik zu kommunizieren. Die Zuleitungsschnittstelle dient insbesondere der Verbindung einer Auswerteeinheit, insbesondere deren Auswerteelektronik, mit dem Motorkopfstück, insbesondere dessen Motorkopfelektronik, vorzugsweise über ein Zuleitungskabel.

Die Motorkopfelektronik kann vorzugsweise ausgebildet sein, um Signale über die Kommunikationsschnittstelle und/oder die Zuleitungsschnittstelle zu senden und/oder zu empfangen. Hierbei kann die Motorkopfelektronik insbesondere ausgebildet sein, um Signale der Kommunikationsschnittstelle in Signale der Zuleitungsschnittstelle umzusetzen. Ferner kann die Adapterelektronik insbesondere ausgebildet sein, um Signale der Zuleitungsschnittstelle in Signale der Kommunikationsschnittstelle umzusetzen.

Ferner bevorzugt ist es, wenn die die Motorkopfelektronik eingerichtet ist, um Signale, die auf einem Busprotokoll basieren, über ein Bussystem oder mehrere Bussysteme an die Kommunikatorelektronik der Kommunikatorstücke zu senden oder von der Kommunikatorelektronik der Kommunikatorstücke zu empfangen. Durch diese Ausgestaltung können sämtliche Kommunikationseinheiten über eine Busleitung mit dem Motorkopfstück elektrisch verbunden sein. Dadurch kann das Motokopfstück Signale der Kommunikationseinheiten über diese eine Busleitung empfangen und/oder Signale an die Kommunikationseinheiten über diese eine Busleitung senden. Vorteilhaft ist ferner, dass die Signale überdie Busleitung zeitgleich übertragen werden können. Es können auch zwei oder mehrere Busleitungen vorgesehen sein, insbesondere auch zwei oder mehrere verschiedene Bussysteme.

In einer Variante kann das Motorkopfstück eingerichtet sein, um über eine Busleitung mit der Auswerteeinheit signaltechnisch gekoppelt zu werden.

In einer Variante kann das Motorkopfstück mit einer Antriebseinheit signaltechnisch koppelbar sein und ausgebildet sein, um die Antriebseinheit zu steuern.

Vorzugsweise kann das Adapterstück und/oder das Kommunikatorstück und/oder das Motorkopfstück eine Speichereinheit aufweisen, die ausgebildet ist, um Signale zu speichern.

Gemäß einer bevorzugten Ausführungsform kann das Adapterstück ein Polymer, einen Kunststoff, ein glasfaserverstärktes Nylon, ein glasfaserverstärktes Acetyl, Aluminium oder eine Aluminiumlegierung umfassen oder aus einem Polymer, einem Kunststoff, einem glasfaserverstärkten Nylon, einem glasfaserverstärktem Acetyl, aus Aluminium oder einer Aluminiumlegierung bestehen.

Vorzugsweise kann das Kommunikatorstück ein Polymer, einen Kunststoff, ein glasfaserverstärktes Nylon, ein glasfaserverstärktes Acetyl, Aluminium oder eine Aluminiumlegierung umfassen oder aus einem Polymer, einem Kunststoff, einem glasfaserverstärkten Nylon, einem glasfaserverstärktem Acetyl, aus Aluminium oder einer Aluminiumlegierung bestehen.

Vorzugsweise kann das Motorkopfstück ein Polymer, einen Kunststoff, ein glasfaserverstärktes Nylon, ein glasfaserverstärktes Acetyl, eloxiertes Metall, Aluminium oder eine Aluminiumlegierung umfassen oder aus einem Polymer, einem Kunststoff, einem glasfaserverstärkten Nylon, einem glasfaserverstärktem Acetyl, eloxiertem Metall, aus Aluminium oder einer Aluminiumlegierung bestehen.

Insbesondere bevorzugt können die Kommunikationseinheiten jeweils ein Feldgerät umfassen, wobei die Adapterelektronik mit der Feldgerätelektronik des Feldgeräts elektrisch verbunden ist.

Für weitere Vorteile, Ausführungsvarianten und Ausführungsdetails dieses dritten Aspekts und dessen mögliche Fortbildungen wird auch auf die zuvor erfolgte Beschreibung zu den entsprechenden Merkmalen und Fortbildungen des ersten Aspekts und des zweiten Aspekts verwiesen.

Gemäß einem weiteren Aspekt der Erfindung wird die eingangs genannte Aufgabe gelöst durch die Verwendung einer Datenübertragungseinheit für eine Kopfplatte und/oder für einen Bioreaktor.

Gemäß einem weiteren Aspekt der Erfindung wird die eingangs genannte Aufgabe gelöst durch ein Verfahren zum Bereitstellen einer Datenübertragungseinheit nach einem der Ansprüche 3 bis 13 den Schritten:
Bereitstellen eines Motorkopfstücks, das mit einer Auswerteeinheit signaltechnisch und mit einer Antriebseinheit, die an einer Kopfplatte eines Bioreaktors anordenbar ist, energietechnisch koppelbar ist, umfassend eine Motorkopfelektronik, und Zusammenstellen von Kommunikationseinheiten, umfassend die Schritte: Auswählen von Adapterstücken mit jeweils einer Adapterelektronik in Abhängigkeit der mit diesen Adapterstücken zu koppelnden Feldgeräte, Bereitstellen von Kommunikatorstücken mit jeweils einer Kommunikatorelektronik, Koppeln der Adapterstücke mit jeweils einem Kommunikatorstück der Kommunikatorstücke und jeweils elektrisches Verbinden der Adapterelektronik mit der Kommunikatorelektronik, jeweils elektrisches Verbinden der Kommunikatorelektronik mit der Motorkopfelektronik.

Durch das modulare System können die Adapterstücke in Abhängigkeit eines Feldgeräts ausgewählt und mit diesem gekoppelt werden. Dieses System umfasst vorzugweise unterschiedliche Gruppen von Adapterstücken, die vorzugsweise entsprechend unterschiedlicher Feldgeräte konfiguriert sind.

Die Kommunikatorstücke können vorzugsweise im Wesentlichen identisch sein und ausgebildet sein, um mit jedem der Adapterstücke gekoppelt zu werden.

Insbesondere kann somit eine Datenübertragungseinheit bereitgestellt werden, die individuell an Kundenbedürfnisse angepasst ist. Vorteilhaft ist ferner, dass eine Datenverarbeitungseinheit auch umgerüstet und/oder aufgerüstet werden kann.

Für weitere Vorteile, Ausführungsvarianten und Ausführungsdetails dieser weiteren Aspekte und deren mögliche Fortbildungen wird auch auf die zuvor erfolgte Beschreibung zu den entsprechenden Merkmalen und Fortbildungen des ersten Aspekts, des zweiten Aspekts und des dritten Aspekts verwiesen.

Bevorzugte Ausführungsbeispiele werden exemplarisch anhand der beiliegenden Figuren erläutert. Es zeigen:
- Fig. 1: einen Ausschnitt eines Bioreaktors;
- Fig. 2: eine Datenübertragungseinheit mit einer Kommunikationseinheit;
- Fig. 3: ein mit einem Feldgerät koppelbaren Adapterstück und einen Ausschnitt eines Feldgeräts; und
- Fig. 4: exemplarische Verfahrensschritte zum Bereitstellen einer Datenübertragungseinheit.

In den Figuren sind gleiche oder im Wesentlichen funktionsgleiche bzw. -ähnliche Elemente mit den gleichen Bezugszeichen bezeichnet.

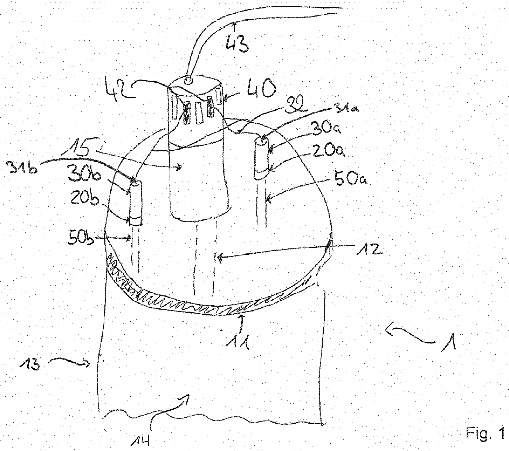

Figur 1 zeigt einen Ausschnitt eines Bioreaktors 1 mit einem Behälter 13 und einer Kopfplatte 11, die den Behälter 13 verschließt. Der Behälter 13 bzw. dessen Wandung und die Kopfplatte 11 umschließen einen Innenraum 14. Der Innenraum 14 kann einen Reaktionsraum des Bioreaktors 1 definieren.

Der Bioreaktor 1 umfasst ferner eine Rührwelle 12, die in den Innenraum 14 geführt ist und an eine Antriebseinheit 15, die an der Kopfplatte 11 angeordnet ist, gekoppelt ist. Mittels der Antriebseinheit 15 kann die Rührwelle 12 und somit ein Rührwerk des Bioreaktors 1 in Rotation versetzt werden, um einen Inhalt, vorzugsweise ein Reaktionsmedium zu durchmischen.

Der in Figur 1 gezeigte Bioreaktor 1 umfasst ferner zwei Feldgeräte 50a, 50b die jeweils mit einer Kommunikationseinheit gekoppelt sind. Die Kommunikationseinheiten sind Bestandteile der Datenübertragungseinheit.

Es sind auch Ausgestaltungen mit nur einem Feldgerät und nur einer Kommunikationseinheit oder mit mehr als zwei Feldgeräten und entsprechend mehr als zwei Kommunikationseinheiten möglich. Ferner sind Ausgestaltungen möglich, in der zwei oder mehr Feldgeräte mit jeweils einem Adapterstück an ein gemeinsames Kommunikatorstück angeschlossen sind.

Die Datenübertragungseinheit umfasst ein Motorkopfstück 40, das auf der Antriebseinheit 15 montiert ist und mit dieser zumindest energietechnisch gekoppelt ist. Das Motorkopfstück 40 ist daher ausgebildet, um die Antriebseinheit 15 mit Energie zu versorgen. Insbesondere kann eine Motorkopfelektronik mit einer Antriebselektronik der Antriebseinheit 40 elektrisch verbunden werden. Vorzugsweise kann das Motorkopfstück 40 ausgebildet sein, um mit der Antriebseinheit 15 signaltechnisch gekoppelt zu werden. Dadurch kann die Motorkopfelektronik vorzugsweise die Antriebseinheit auch ansteuern.

An das Motorkopfstück 40 ist ein Zuleitungskabel 43 angeschlossen. Dieses Zuleitungskabel 43 kann vorzugsweise mindestens vier Adern, zwei zur Energieversorgung und zwei zur Datenübertragung, umfassen Über das Zuleitungskabel 43 kann das Motorkopfstück 40 mit einer Auswerteeinheit elektrisch verbunden werden. Die signalführenden Adern können beispielweise eine Datenübertragung nach dem EIA485/RS485 Standard ermöglichen. Durch die Nutzung einer differentiell basierten Übertragungstechnologie kann eine besonders robuste und für Fehler unanfällige Kommunikation ermöglicht werden.

Die Motorkopfelektronik kann vorzugsweise ausgebildet sein, um Signale über beispielsweise Modbus zur Auswerteeinheit zu senden oder von dieser zu empfangen. Die Motorkopfelektronik kann ferner ausgebildet sein, um im Bridge-Betrieb zwischen den Feldgeräten und der Auswerteeinheit bzw. einer Steuereinheit und zusätzlich als Hub für Feldgeräte an einem Bioreaktor zu arbeiten.

Die Auswerteeinheit kann vorzugsweise ausgebildet sein, um Signale, vorzugsweise von der Datenübertragungseinheit, zu empfangen und diese auszuwerten. Ferner kann die Auswerteeinheit ausgebildet sein, um Signale zu erzeugen und an die Datenübertragungseinheit zu senden. Vorzugsweise können diese Signale dazu dienen, die Feldgeräte 50a, 50b anzusteuern und/oder zu kalibrieren. Insbesondere bevorzugt, kann die Auswerteeinheit Signale von der Datenübertragungseinheit empfangen, diese Signale auswerten und in Abhängigkeit dieser Signale Signale erzeugen und an die Datenübertragungseinheit senden.

Die Kommunikationseinheiten umfassen jeweils ein Adapterstück 20a, 20b und jeweils ein Kommunikatorstück 30a, 30b. Das Adapterstück 20a, 20b umfasst eine Adapterelektronik, die mit der Feldgerätelektronik eines der Feldgeräte 50a, 50b elektrisch verbunden ist. Das Kommunikatorstück 20a, 20b umfasst eine Kommunikatorelektronik, die mit der Adapterelektronik und der Motorkopfelektronik des Motorkopfs 40 elektrisch verbunden ist.

Das in Figur 1 gezeigte Motorkopfstück 40 ist kabelgebunden mit den Kommunikatorstücken 30a, 30b verbunden. Hierzu weist das Motorkopfstück 40 Buchsen 42 auf, die jeweils als eine Kommunikationsschnittstelle ausgebildet ist. Die Motorkopfschnittstellen 31a, 31b des Kommunikatorstücks 30a, 30b sind jeweils als ein Anschluss an einem Kabel 32 ausgebildet, wobei das Kabel 32 an der Motorkopfschnittstellen 31a, 31b fest oder lösbar angebracht sein kann. Der Anschluss ist hierbei als ein Stecker ausgebildet, der eingerichtet ist, um an Kontakten der Buchse 42 am Motorkopfstück 40 angeschlossen zu werden.

Durch diese Ausgestaltung können Signale zwischen den Feldgeräten 50a, 50b und den Adapterstücken 20a, 20b und zwischen den Adapterstücken 20a, 20b und den Kommunikatorstücken 30a, 30b und zwischen den Kommunikatorstücken 30a, 30b und dem Motorkopfstück 40 kommuniziert werden. Die in Figur 1 nicht dargestellten Adapterelektroniken umfassen jeweils einen Signalwandler, um die Signale des Feldgeräts 50a, 50b umzusetzen, vorzugsweise in standardisierte Signale, und diese an eine Kommunikatorelektronik des Kommunikatorstücks 30a, 30b zu senden. Vorzugsweise kann die Adapterelektronik ausgebildet sein, um Signale von der Kommunikatorelektronik zu empfangen, mittels des Signalwandlers umzusetzen und an eine Feldgerätelektronik zu senden.

Die Feldgeräte 50a, 50b, die Adapterstücke 20a, 20b, die Kommunikatorstücke 30a, 30b und das Motorkopfstück 40 sind separate Bauteile, die miteinander gekoppelt sind.

Durch signaltechnisches Koppeln des Motorkopfstücks 40 mit der Auswerteeinheit und jeweils des Adapterstücks 20a, 20b mit dem Feldgerät 50a, 50b kann mittels der Datenübertragungseinheit eine Signalübertragung zwischen mindestens zwei Feldgeräten 50a, 50b und der Auswerteeinheit ermöglicht werden.

Unterschiedliche Feldgeräte 50a, 50b sind in der Regel eingerichtet, um unterschiedliche Signale, die Feldgerätinformationen und Messinformationen tragen, zu senden und/oder unterschiedliche Signale, die Steuerinformationen tragen, zu empfangen. Beispielsweise können Feldgeräte 50a, 50b digitale oder analoge Signale senden und/oder empfangen. Insbesondere können Feldgeräte 50a, 50b analoge Signale erzeugen. Aus diesem Grund ist es notwendig entsprechend konfigurierte Adapterstücke 20a, 20b einzusetzen, um diese Signale umsetzen und an das Kommunikatorstück 30a, 30b senden zu können und/oder diese Signale von dem Kommunikatorstück 30a, 30b empfangen, umsetzen und an das Feldgerät 501, 50b senden zu können.

Das Kommunikatorstück 30a, 30b kann vorzugsweise im Gegensatz zu dem Adapterstück 20a, 20b für jede Kommunikationseinheit gleich ausgebildet sein. Die Kommunikatorstücke 30a, 30b können demnach vorzugsweise einheitlich konfiguriert sein. Eine Kommunikatorelektronik jedes Kommunikatorstücks 30a, 30b ist mit der Motorkopfelektronik elektrisch verbunden, um Signale des Feldgeräts 50a, 50b über das Adapterstück 20a, 20b zu empfangen und diese an die Motorkopfelektronik zu senden und/oder Signale von der Motorkopfelektronik zu empfangen und über das Adapterstück 20a, 20b an das Feldgerät 50a, 50b zu senden. Dadurch kann die Motorkopfelektronik Signale der einzelnen Feldgeräte 50a, 50b empfangen und/oder Signale an diese senden.

Durch unterschiedliche Adapterstücke 20a, 20b, die entsprechend der Feldgeräte 50a, 50b konfiguriert sind, kann der Bioreaktor 1, insbesondere die Datenübertragungseinheit, individuell an Kundenbedürfnisse angepasst werden. Dadurch muss auf Hardwareseite nur ein entsprechend konfiguriertes Adapterstück 20a, 20b entworfen werden - der Aufwand ein neues Feldgerät 50a, 50b in den Bioreaktor 1 zu integrieren ist also deutlich geringer.

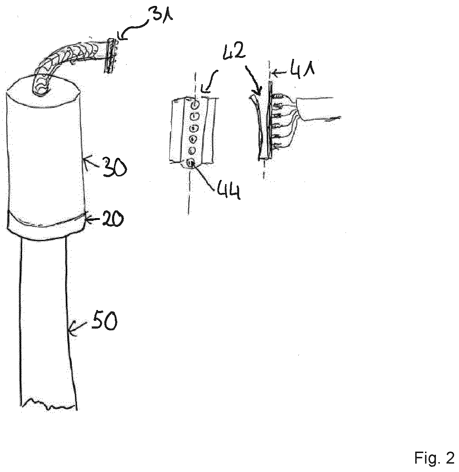

Figur 2 zeigt links eine mit einem Feldgerät 50 gekoppelte Kommunikationseinheit mit einem Adapterstück 20 und einem Kommunikatorstück 30. Gemäß dem hier gezeigten Ausführungsbeispiel ist das Adapterstück 20 direkt auf das Feldgerät 50 montiert und mit diesem elektrisch verbunden. Gleichermaßen ist das Kommunikatorstück 30 direkt auf das Adapterstück 20 montiert und mit diesem verbunden. Das Kommunikatorstück 30 weist eine Motorkopfschnittstelle 31 auf, die als ein Anschluss an einem Kabel ausgebildet ist. Der Anschluss ist hierbei als ein Stecker ausgebildet, der eingerichtet ist, um an Kontakten 44 einer Buchse 42 am Motorkopfstück angeschlossen zu werden. Die Buchse 42 ist an einem Gehäuse 41 des Motorkopfstücks ausgebildet.

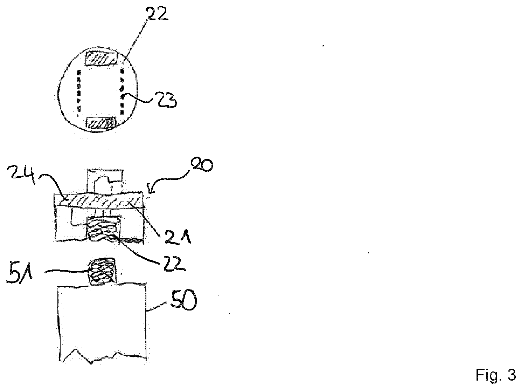

Figur 3 zeigt ein Feldgerät 50 mit einer Anschlussschnittstelle 51, die mit einer Feldgerätschnittstelle 22 des Adapterstücks 20 koppelbar ist. Die Anschlussschnittstelle 51 und die Feldgerätschnittstelle 22 ermöglichen eine Ankopplung des Feldgeräts 50 an das Adapterstück 20. Die in Figur 3 nicht dargestellte Adapterelektronik ist auf einer Leiterplatte, einem PCBA, 24 untergebracht.

Das Adapterstück 20 weist eine Feldgerätschnittstelle 22 auf, die als Pins 23 auf der Leiterplatte 24 ausgebildet ist. Die Leiterplatte weist gemäß dem hier gezeigten Beispiel insgesamt sechs Pin-Paare auf. Hiervon können beispielsweise mindestens zwei Pins konfiguriert sein, um eine elektrische Verbindung zwischen der Adapterelektronik und der Feldgerätelektronik zu ermöglichen, wobei diese Pins 23 vorzugsweise als Ausgänge und/oder Eingänge eingerichtet sind. Darüber hinaus kann die Leiterplatte 24 vorzugsweise zwei Pins 23 aufweisen, die zur Energieversorgung eingerichtet sind. Die Leiterplatte kann insbesondere zwei Federkontakte aufweisen, die eine elektrische Verbindung zwischen der Adapterelektronik und der Kommunikatorelektronik ermöglichen. Die Leiterplatte könnte beispielsweise folgende Musterbelegung haben: + 24 V, GND, RS485+, RS485-, Analog1, Analog2.

Die Pins 23 können vorzugsweise eine partielle Metallisierung der Kontaktflächen, insbesondere eine Vergoldung, aufweisen.

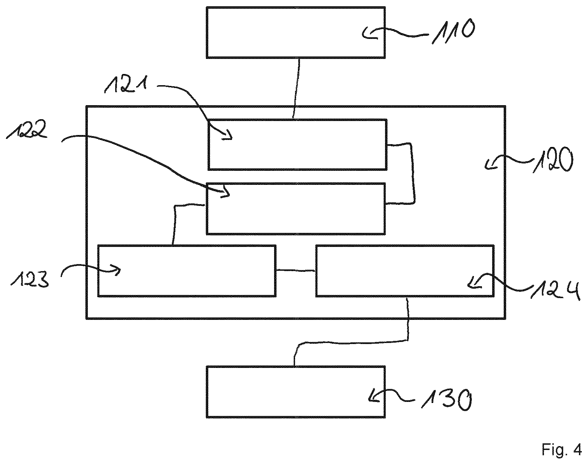

Figur 4 zeigt beispielhafte Verfahrensschritte zum Bereitstellen einer Datenübertragungseinheit. Hierbei wird in einem Schritt 110 ein Motorkopfstück, das mit einer Auswerteeinheit signaltechnisch und mit einer Antriebseinheit, die an einer Kopfplatte eines Bioreaktors anordenbar ist, energietechnisch koppelbar ist, umfassend eine Motorkopfelektronik, ausgewählt bzw. bereitgestellt. In einem weiteren Schritt 120 werden Kommunikationseinheiten zusammengestellt. Der Schritt 120 des Zusammenstellens der Kommunikationseinheiten umfasst ferner einen Schritt 121 des Auswählens von Adapterstücken mit jeweils einer Adapterelektronik in Abhängigkeit der mit diesen Adapterstücken zu koppelnden Feldgeräte, und einen Schritt 122 des Bereitstellens von Kommunikatorstücken mit jeweils einer Kommunikatorelektronik, sowie einen Schritt 123 des Koppelns der Adapterstücke mit jeweils einem Kommunikatorstück der Kommunikatorstücke und jeweils einen Schritt 124 des elektrisches Verbindens der Adapterelektronik mit der Kommunikatorelektronik. Ferner werden die Kommunikatorelektroniken in einem Schritt 130 mit der Motorkopfelektronik elektrisch verbunden.

### Bezugszeichenliste

- 1: Bioreaktor
- 11: Kopfplatte
- 12: Rührwelle
- 13: Behälter
- 14: Innenraum des Behälters
- 15: Antriebseinheit
- 20: Adapterstück
- 20a: erstes Adapterstück
- 20b: zweites Adapterstück
- 21: Leiterplatte / PCBA
- 22: Feldgerätschnittstelle
- 23: Pins
- 24: Leiterplatte / PCBA
- 30: Kommunikatorstück
- 30a: erstes Kommunikatorstück
- 30b: zweites Kommunikatorstück
- 31: Motorkopfschnittstelle
- 31a: Motorkopfschnittstelle
- 31b: Motorkopfschnittstelle
- 32: Kabel
- 40: Motorkopfstück
- 41: Gehäuse des Motorkopfstücks
- 42: Buchse am Motorkopfstück
- 43: Zuleitungskabel
- 44: Kontakte an der Buchse
- 50: Feldgerät / Sensor / Aktor
- 50a: erstes Feldgerät / erster Sensor / erster Aktor
- 50b: zweites Feldgerät / zweiter Sensor / zweiter Aktor
- 51: Anschlussschnittstelle
- 110: Auswählen eines Motorkopfstücks
- 120: Zusammenstellen von Kommunikationseinheiten
- 121: Auswählen von Adapterstücken
- 122: Bereitstellen von Kommunikatorstücken
- 123: Koppeln der Adapterstücke
- 124: elektrisches Verbinden der Adapterelektronik mit der Kommunikatorelektronik
- 130: elektrisches Verbinden der Kommunikatorelektronik mit der Motorkopfelektronik

## Patentansprüche

1. Bioreaktor (1), insbesondere zur Kultivierung von Mikroorganismen und/oder Zellkulturen, mit
- einem Behälter (13),
- einer Kopfplatte (11), die ausgebildet ist, um den Behälter (13) zu verschließen,
- einem Rührwerk mit
∘ einer durch die Kopfplatte (11), in einen Innenraum (14) des Behälters (13) geführten Rührwelle (12) und
∘ einem in dem Innenraum (14) des Behälters (13) mit der Rührwelle (12) verbundenen Rührelement,
- einer an der Kopfplatte (11) angeordneten, mit der Rührwelle (12) gekoppelten Antriebseinheit (15) und
- mindestens einem in dem Innenraum (14) angeordneten Feldgerät (50, 50a, 50b) mit einer Feldgerätelektronik,
wobei die Kopfplatte (11) umfasst:
- eine Innenseite und eine der Innenseite gegenüberliegende Außenseite,
- eine Rührwerksanschlusseinrichtung, die ausgebildet ist, um
∘ die Rührwelle (12) durch die Kopfplatte (11) hindurchzuführen und
∘ die Antriebseinheit (15) anzuschließen,
- mindestens einer Feldgerätanschlusseinrichtung, die ausgebildet ist, um das Feldgerät (50, 50a, 50b) an der Innenseite anzuschließen, und
- eine an der Außenseite angeordnete Datenübertragungseinheit, mit
∘ einem Motorkopfstück (40), das mit einer Auswerteeinheit signaltechnisch koppelbar und mit der Antriebseinheit (15) energietechnisch gekoppelt ist, umfassend eine Motorkopfelektronik, und
∘ mindestens einer mit dem Motorkopfstück (40) signaltechnisch gekoppelten Kommunikationseinheit, umfassend:
▪ ein Adapterstück (20, 20a, 20b) mit einer Adapterelektronik, die mit der Feldgerätelektronik des Feldgeräts (50, 50a, 50b) elektrisch verbunden ist, und
▪ ein Kommunikatorstück (30, 30a, 30b) mit einer Kommunikatorelektronik, die mit der Adapterelektronik und der Motorkopfelektronik elektrisch verbunden ist,
wobei die Adapterelektronik eingerichtet ist, um Signale zwischen der Feldgerätelektronik und der Kommunikatorelektronik zu kommunizieren, wobei die Signale Feldgerätinformationen und Messinformationen und/oder Steuerinformation tragen,
wobei die Kommunikatorelektronik eingerichtet ist, um die Signale zwischen der Adapterelektronik und der Motorkopfelektronik zu kommunizieren,
wobei die Datenübertragungseinheit ferner einen Signalwandler umfasst, der vorzugsweise als Teil der Adapterelektronik und/oder als Teil der Kommunikatorelektronik ausgebildet ist;
wobei das Adapterstück (20, 20a, 20b) und das Kommunikatorstück (30, 30a, 30b) als separate, miteinander koppelbare Bauteile ausgebildet sind,
wobei das Motorkopfstück (40) als ein separates, mit der Kommunikationseinheit koppelbares Bauteil ausgebildet ist.

2. Kopfplatte (11) zum Verschließen eines Bioreaktors (1), umfassend
- eine Innenseite und eine der Innenseite gegenüberliegende Außenseite,
- eine Rührwerksanschlusseinrichtung, die ausgebildet ist, um
∘ eine Rührwelle (12) durch die Kopfplatte (11) hindurchzuführen und
∘ eine mit der Rührwelle (12) koppelbare Antriebseinheit (15) anzuschließen,
- mindestens eine Feldgerätanschlusseinrichtung, die ausgebildet ist, um ein Feldgerät (50, 50a, 50b) an der Innenseite anzuschließen,
- eine an der Außenseite angeordnete Datenübertragungseinheit, mit
∘ einem Motorkopfstück (40), das mit einer Auswerteeinheit signaltechnisch und mit der Antriebseinheit (15), die an einer Kopfplatte (11) eines Bioreaktors (1) anordenbar ist, energietechnisch koppelbar ist, umfassend eine Motorkopfelektronik, und
∘ mindestens einer mit dem Motorkopfstück (40) signaltechnisch gekoppelten Kommunikationseinheit, umfassend:
▪ ein Adapterstück (20, 20a, 20b) mit einer Adapterelektronik, die mit einer Feldgerätelektronik des Feldgeräts (50, 50a, 50b) elektrisch verbindbar ist, und
▪ ein Kommunikatorstück (30, 30a, 30b) mit einer Kommunikatorelektronik, die mit der Adapterelektronik und der Motorkopfelektronik elektrisch verbunden ist,
wobei die Adapterelektronik eingerichtet ist, um Signale zwischen der Feldgerätelektronik und der Kommunikatorelektronik zu kommunizieren, wobei die Signale Feldgerätinformationen und Messinformationen und/oder Steuerinformation tragen,
wobei die Kommunikatorelektronik eingerichtet ist, um die Signale zwischen der Adapterelektronik und der Motorkopfelektronik zu kommunizieren,
wobei die Datenübertragungseinheit ferner einen Signalwandler umfasst, der vorzugsweise als Teil der Adapterelektronik und/oder als Teil der Kommunikatorelektronik ausgebildet ist,
wobei das Adapterstück (20, 20a, 20b) und das Kommunikatorstück (30, 30a, 30b) als separate, miteinander koppelbare Bauteile ausgebildet sind,
wobei das Motorkopfstück (40) als ein separates, mit der Kommunikationseinheit koppelbares Bauteil ausgebildet ist.

3. Datenübertragungseinheit für eine Kopfplatte (11) eines Bioreaktors (1) mit
- einem Motorkopfstück (40), das mit einer Auswerteeinheit signaltechnisch und mit einer Antriebseinheit (15), die an einer Kopfplatte (11) eines Bioreaktors (1) anordenbar ist, energietechnisch koppelbar ist, umfassend eine Motorkopfelektronik, und
- mindestens eine mit dem Motorkopfstück (40) signaltechnisch gekoppelte Kommunikationseinheit, jeweils umfassend:
∘ ein Adapterstück (20, 20a, 20b) mit einer Adapterelektronik, die mit einer Feldgerätelektronik eines Feldgeräts (50, 50a, 50b) elektrisch verbindbar ist, und
∘ ein Kommunikatorstück (30, 30a, 30b) mit einer Kommunikatorelektronik, die mit der Adapterelektronik und der Motorkopfelektronik elektrisch verbunden ist,
wobei die Adapterelektronik eingerichtet ist, um Signale zwischen der Feldgerätelektronik und der Kommunikatorelektronik zu kommunizieren, wobei die Signale Feldgerätinformationen und Messinformationen und/oder Steuerinformation tragen,
wobei die Kommunikatorelektronik eingerichtet ist, um die Signale zwischen der Adapterelektronik und der Motorkopfelektronik zu kommunizieren,
wobei die Datenübertragungseinheit ferner einen Signalwandler umfasst, der vorzugsweise als Teil der Adapterelektronik und/oder als Teil der Kommunikatorelektronik ausgebildet ist,
wobei das Adapterstück (20, 20a, 20b) und das Kommunikatorstück (30, 30a, 30b) als separate, miteinander koppelbare Bauteile ausgebildet sind,
wobei das Motorkopfstück (40) als ein separates, mit den Kommunikationseinheiten koppelbares Bauteil ausgebildet ist.

4. Datenübertragungseinheit nach mindestens Anspruch 3, wobei
- das Adapterstück (20, 20a, 20b) eine Feldgerätschnittstelle und eine Kommunikatorschnittstelle umfasst, wobei die Adapterelektronik
∘ zwischen die Feldgerätschnittstelle und die Kommunikatorschnittstelle geschaltet ist,
∘ über die Feldgerätschnittstelle mit der Feldgerätelektronik elektrisch verbindbar ist und
∘ über die Kommunikatorschnittstelle mit der Kommunikatorelektronik elektrisch verbunden ist, und
- das Kommunikatorstück (30, 30a, 30b) eine Adapterschnittstelle und eine Motorkopfschnittstelle umfasst, wobei die Kommunikatorelektronik
∘ zwischen die Adapterschnittstelle und die Motorkopfschnittstelle geschaltet ist,
∘ über die Adapterschnittstelle mit der Adapterelektronik elektrisch verbunden ist und
∘ über die Motorkopfschnittstelle mit der Motorkopfelektronik elektrisch verbunden ist.

5. Datenübertragungseinheit nach mindestens einem der Ansprüche 3 oder 4, umfassend mindestens eine erste Gruppe von ersten Adapterstücken und eine zweite Gruppe von zweiten Adapterstücken,
wobei die Adapterelektronik der ersten Adapterstücke (20a) ausgebildet ist, um die Signale zwischen der Feldgerätelektronik eines ersten Feldgeräts (50a) und der Kommunikatorelektronik zu kommunizieren,
wobei die Adapterelektronik der zweiten Adapterstücke (20b) ausgebildet ist, um die Signale zwischen der Feldgerätelektronik eines zweiten Feldgeräts (50b) und der Kommunikatorelektronik zu kommunizieren,
wobei das Adapterstück (20, 20a, 20b) jeder Kommunikationseinheit in Abhängigkeit eines zu koppelnden Feldgeräts (50, 50a, 50b) ausgewählt ist aus mindestens der ersten Gruppe von ersten Adapterstücken (50a) und der zweiten Gruppe von zweiten Adapterstücken (50b).

6. Datenübertragungseinheit nach mindestens einem der Ansprüche 3 bis 5, wobei
- das Adapterstück (20, 20a, 20b) eine Feldgerätkupplung umfasst, die ausgebildet ist, um das Feldgerät (50, 50a, 50b) mit dem Adapterstück (20, 20a, 20b) mechanisch, vorzugsweise lösbar, zu verbinden, und/oder
- das das Kommunikatorstück (30, 30a, 30b) eine Adapterkupplung umfasst, die ausgebildet ist, um das Adapterstück (20, 20a, 20b) mit dem Kommunikatorstück (30, 30a, 30b) mechanisch, vorzugsweise lösbar, zu verbinden.

7. Datenübertragungseinheit nach mindestens einem der Ansprüche 3 bis 6,
- wobei der Signalwandler ein Analog-Digital-Umsetzer ist,
und/oder
- wobei die Adapterelektronik und/oder die Kommunikatorelektronik und/oder der Signalwandler eingerichtet ist, um analoge Signale zu empfangen, in normierte analoge Signale umzuwandeln und die normierten analogen Signale in digitale Signale umzusetzen und die digitalen Signale zu senden und/oder umgekehrt.

8. Datenübertragungseinheit nach mindestens einem der Ansprüche 3 bis 7, wobei die Kommunikatorelektronik und/oder die Motorkopfelektronik dazu eingerichtet ist, die Signale kontaktlos oder kontaktgebunden, vorzugsweise kabelgebunden, zu empfangen, die Signale in Funksignale umzusetzen und per Funk oder kabelgebunden zu senden und/oder umgekehrt.

9. Datenübertragungseinheit nach mindestens einem der Ansprüche 3 bis 8, wobei die Adapterelektronik dazu eingerichtet ist, die Signale kontaktlos oder kontaktgebunden, vorzugsweise kabelgebunden, zu empfangen, die Signale in Funksignale umzusetzen und per Funk oder kabelgebunden zu senden und/oder umgekehrt.

10. Datenübertragungseinheit nach mindestens Anspruch 4 und einem der Ansprüche 5 bis 9, wobei die Feldgerätschnittstelle und/oder die Kommunikatorschnittstelle und/oder die Adapterschnittstelle und/oder die Motorkopfschnittstelle als ein Anschluss an einem Kabel ausgebildet ist, wobei vorzugsweise das Kabel eine Länge von maximal 50 cm aufweist.

11. Datenübertragungseinheit nach mindestens einem der Ansprüche 3 bis 10, wobei
- das Motorkopfstück (40) mit dem Kommunikatorstück (30, 30a, 30b) energietechnisch gekoppelt ist, wobei die Motorkopfelektronik eingerichtet ist, um die Kommunikatorelektronik mit Strom zu versorgen, und/oder
- das Kommunikatorstück (30, 30a, 30b) mit dem Adapterstück (20, 20a, 20b) energietechnisch gekoppelt ist, wobei die Kommunikatorelektronik eingerichtet ist, um die Adapterelektronik mit Strom zu versorgen, und/oder
- das Adapterstück (20, 20a, 20b) mit dem Feldgerät (50, 50a, 50b) energietechnisch koppelbar ist, wobei die Adapterelektronik eingerichtet ist, um die Feldgerätelektronik mit Strom zu versorgen,
wobei vorzugsweise der Strom kontaktgebunden, vorzugsweise kabelgebunden, und/oder induktiv übertragen wird.

12. Datenübertragungseinheit nach mindestens einem der Ansprüche 3 bis 11, wobei das Motorkopfstück (40) umfasst:
- eine Kommunikationsschnittstelle, die mit der Kommunikatorelektronik elektrisch verbunden ist, und
- eine Zuleitungsschnittstelle, an die ein Zuleitungskabel elektrisch anschließbar ist, wobei die Zuleitungsschnittstelle mit einer Auswerteelektronik der Auswerteeinheit elektrisch verbindbar ist,
wobei die Motorkopfelektronik zwischen die Kommunikationsschnittstelle und die Zuleitungsschnittstelle geschaltet ist,
wobei die Motorkopfelektronik eingerichtet ist, um Signale zwischen der mit der Kommunikationsschnittstelle elektrisch verbundenen Kommunikatorelektronik und einer mit der Zuleitungsschnittstelle elektrisch verbindbaren Auswerteelektronik zu kommunizieren.

13. Datenübertragungseinheit nach mindestens einem der Ansprüche 3 bis 12, wobei die Motorkopfelektronik eingerichtet ist, um Signale, die auf einem Busprotokoll basieren, über ein Bussystem oder mehrere Bussysteme an die Kommunikatorelektronik der Kommunikatorstücke (30, 30a, 30b) zu senden oder von der Kommunikatorelektronik der Kommunikatorstücke (30, 30a, 30b) zu empfangen.

14. Verwendung einer Datenübertragungseinheit nach einem der Ansprüche 3 bis 13 für eine Kopfplatte (11), insbesondere nach mindestens Anspruch 2, und/oder für einen Bioreaktor (1), insbesondere nach mindestens Anspruch 1.

15. Verfahren zum Bereitstellen einer Datenübertragungseinheit nach einem der Ansprüche 3 bis 13 mit den Schritten:
- Bereitstellen eines Motorkopfstücks (110), das mit einer Auswerteeinheit signaltechnisch und mit einer Antriebseinheit, die an einer Kopfplatte eines Bioreaktors anordenbar ist, energietechnisch koppelbar ist, umfassend eine Motorkopfelektronik, und
- Zusammenstellen von mindestens einer Kommunikationseinheit (120), umfassend die Schritte:
∘ Auswählen eines Adapterstücks (121) mit einer Adapterelektronik in Abhängigkeit des mit diesem Adapterstück zu koppelnden Feldgeräts,
∘ Bereitstellen eines Kommunikatorstücks (122) mit einer Kommunikatorelektronik,
∘ Koppeln des Adapterstücke (123) mit dem Kommunikatorstück und elektrisches Verbinden der Adapterelektronik mit der Kommunikatorelektronik (124),
- elektrisches Verbinden der Kommunikatorelektronik mit der Motorkopfelektronik (130).

## Claims

1. A bioreactor (1), in particular for the cultivation of microorganisms and/or cell cultures, comprising
- a container (13),
- a head plate (11) designed to seal the container (13),
- a stirring mechanism comprising
∘ a stirring shaft (12) guided through the head plate (11) into an interior (14) of the vessel (13), and
∘ a stirring element connected to the stirring shaft (12) within the interior (14) of the vessel (13),
- a drive unit (15) arranged on the head plate (11) and coupled to the stirring shaft (12), and
- at least one field device (50, 50a, 50b) arranged in the interior (14) and comprising field device electronics,
wherein the head plate (11) comprises:
- an inner side and an outer side opposite the inner side,
- a stirrer connection device configured to
∘ guide the stirring shaft (12) through the head plate (11) and
∘ connect the drive unit (15),
- at least one field device connection means configured to connect the field device (50, 50a, 50b) to the inner side, and
- a data transmission unit arranged on the outer side, comprising
∘ a motor head assembly (40) that is signal-coupled to an evaluation unit and power-coupled to the drive unit (15), comprising motor head electronics, and
∘ at least one communication unit electrically coupled to the motor head unit (40), comprising:
▪ an adapter unit (20, 20a, 20b) with adapter electronics that are electrically connected to the field device electronics of the field device (50, 50a, 50b), and
▪ a communicator unit (30, 30a, 30b) with communicator electronics that are electrically connected to the adapter electronics and the motor head electronics,
wherein the adapter electronics are configured to transmit signals between the field device electronics and the communicator electronics, wherein the signals carry field device information and measurement information and/or control information,
wherein the communicator electronics are configured to transmit the signals between the adapter electronics and the motor head electronics,
wherein the data transmission unit further comprises a signal converter, which is preferably formed as part of the adapter electronics and/or as part of the communicator electronics;
wherein the adapter unit (20, 20a, 20b) and the communicator unit (30, 30a, 30b) are configured as separate, interconnectable components,
wherein the motor headpiece (40) is configured as a separate component that can be coupled to the communication unit.

2. Head plate (11) for sealing a bioreactor (1), comprising
- an inner side and an outer side opposite the inner side,
- a stirrer connection device configured to
∘ guide a stirrer shaft (12) through the head plate (11) and
∘ connect a drive unit (15) that can be coupled to the stirrer shaft (12),
- at least one field device connection means configured to connect a field device (50, 50a, 50b) to the inner side,
- a data transmission unit arranged on the outside, comprising
∘ a motor head assembly (40) that can be signal-coupled to an evaluation unit and power-coupled to the drive unit (15), which can be arranged on a head plate (11) of a bioreactor (1), comprising motor head electronics, and
∘ at least one communication unit electrically coupled to the motor head unit (40), comprising:
▪ an adapter unit (20, 20a, 20b) with adapter electronics that can be electrically connected to field device electronics of the field device (50, 50a, 50b), and
▪ a communicator unit (30, 30a, 30b) with communicator electronics that are electrically connected to the adapter electronics and the motor head electronics,
wherein the adapter electronics are configured to transmit signals between the field device electronics and the communicator electronics, wherein the signals carry field device information and measurement information and/or control information,
wherein the communicator electronics are configured to transmit the signals between the adapter electronics and the motor head electronics,
wherein the data transmission unit further comprises a signal converter, which is preferably formed as part of the adapter electronics and/or as part of the communicator electronics,
wherein the adapter unit (20, 20a, 20b) and the communicator unit (30, 30a, 30b) are configured as separate, interconnectable components,
wherein the motor headpiece (40) is configured as a separate component that can be coupled to the communication unit.

3. Data transmission unit for a head plate (11) of a bioreactor (1) comprising
- a motor headpiece (40) that can be coupled signal-technically to an evaluation unit and power-technically to a drive unit (15) that can be arranged on a head plate (11) of a bioreactor (1), comprising motor head electronics, and
- at least one communication unit coupled to the motor head assembly (40) via signal transmission, each comprising:
∘ an adapter unit (20, 20a, 20b) with adapter electronics that can be electrically connected to the field device electronics of a field device (50, 50a, 50b), and
∘ a communicator unit (30, 30a, 30b) with communicator electronics that are electrically connected to the adapter electronics and the motor head electronics,
wherein the adapter electronics are configured to transmit signals between the field device electronics and the communicator electronics, wherein the signals carry field device information and measurement information and/or control information,
wherein the communicator electronics are configured to transmit the signals between the adapter electronics and the engine head electronics, wherein the data transmission unit further comprises a signal converter, which is preferably formed as part of the adapter electronics and/or as part of the communicator electronics,
wherein the adapter unit (20, 20a, 20b) and the communicator unit (30, 30a, 30b) are configured as separate components that can be coupled to one another,
wherein the motor head piece (40) is configured as a separate component that can be coupled to the communication units.

4. Data transmission unit according to at least claim 3, wherein
- the adapter unit (20, 20a, 20b) comprises a field device interface and a communicator interface, wherein the adapter electronics
∘ is connected between the field device interface and the communicator interface,
∘ can be electrically connected to the field device electronics via the field device interface, and
∘ is electrically connected to the communicator electronics via the communicator interface, and
- the communicator unit (30, 30a, 30b) comprises an adapter interface and a motor head interface, wherein the communicator electronics
∘ is connected between the adapter interface and the motor head interface,
∘ is electrically connected to the adapter electronics via the adapter interface, and
∘ is electrically connected to the motor head electronics via the motor head interface.

5. A data transmission unit according to at least one of claims 3 or 4, comprising at least a first group of first adapter pieces and a second group of second adapter pieces,
wherein the adapter electronics of the first adapter pieces (20a) are configured to transmit the signals between the field device electronics of a first field device (50a) and the communicator electronics,
wherein the adapter electronics of the second adapter pieces (20b) are configured to transmit the signals between the field device electronics of a second field device (50b) and the communicator electronics,
wherein the adapter module (20, 20a, 20b) of each communication unit is selected, depending on a field device (50, 50a, 50b) to be coupled, from at least the first group of first adapter modules (50a) and the second group of second adapter modules (50b).

6. A data transmission unit according to at least one of claims 3 to 5, wherein
- the adapter piece (20, 20a, 20b) comprises a field device coupling configured to mechanically connect the field device (50, 50a, 50b) to the adapter piece (20, 20a, 20b), preferably in a detachable manner, and/or
- the communicator piece (30, 30a, 30b) comprises an adapter coupling configured to mechanically connect the adapter piece (20, 20a, 20b) to the communicator piece (30, 30a, 30b), preferably in a detachable manner.

7. A data transmission unit according to at least one of claims 3 through 6,
- wherein the signal converter is an analog-to-digital converter,
and/or
- wherein the adapter electronics and/or the communicator electronics and/or the signal converter is configured to receive analog signals, convert them into standardized analog signals, convert the standardized analog signals into digital signals, and transmit the digital signals, and/or vice versa.

8. Data transmission unit according to at least one of claims 3 to 7, wherein the communicator electronics and/or the engine head electronics are configured to receive the signals contactlessly or via a connection, preferably via a cable, to convert the signals into radio signals, and to transmit them via radio or via a cable and/or vice versa.

9. Data transmission unit according to at least one of claims 3 to 8, wherein the adapter electronics are configured to receive the signals contactlessly or via a physical connection, preferably via a cable, to convert the signals into radio signals, and to transmit them via radio or via a cable and/or vice versa.

10. Data transmission unit according to at least claim 4 and one of claims 5 to 9, wherein the field device interface and/or the communicator interface and/or the adapter interface and/or the motor head interface is configured as a connector on a cable, wherein the cable preferably has a maximum length of 50 cm.

11. Data transmission unit according to at least one of claims 3 to 10, wherein
- the motor head unit (40) is electrically coupled to the communicator unit (30, 30a, 30b), wherein the motor head electronics are configured to supply power to the communicator electronics, and/or
- the communicator unit (30, 30a, 30b) is electrically coupled to the adapter unit (20, 20a, 20b), wherein the communicator electronics are configured to supply power to the adapter electronics, and/or
- the adapter unit (20, 20a, 20b) is electrically connectable to the field device (50, 50a, 50b), wherein the adapter electronics are configured to supply power to the field device electronics,
wherein the power is preferably transmitted via a contact connection, preferably via a cable, and/or inductively.

12. Data transmission unit according to at least one of claims 3 through 11, wherein the motor head (40) comprises:
- a communication interface that is electrically connected to the communicator electronics, and
- a supply line interface to which a supply line cable can be electrically connected, wherein the supply line interface can be electrically connected to evaluation electronics of the evaluation unit,
wherein the motor head electronics are connected between the communication interface and the supply line interface,
wherein the motor head electronics are configured to transmit signals between the communicator electronics, which are electrically connected to the communication interface, and evaluation electronics that can be electrically connected to the supply line interface via a bus system or multiple bus systems ( ).

13. Data transmission unit according to at least one of claims 3 through 12, wherein the engine head electronics are configured to transmit signals based on a bus protocol via one or more bus systems to the communicator electronics of the communicator units (30, 30a, 30b) via a bus system or multiple bus systems, or to receive such signals from the communicator electronics of the communicator pieces (30, 30a, 30b).

14. Use of a data transmission unit according to any one of claims 3 to 13 for a head plate (11), in particular according to at least claim 2, and/or for a bioreactor (1), in particular according to at least claim 1.

15. A method for providing a data transmission unit according to any one of claims 3 to 13, comprising the steps of:
- providing a motor headpiece (110) that can be coupled signal-technically to an evaluation unit and power-technically to a drive unit, which can be arranged on a head plate of a bioreactor, comprising motor head electronics, and
- assembling at least one communication unit (120), comprising the steps of:
∘ selecting an adapter unit (121) with adapter electronics depending on the field device to be coupled to this adapter unit,
∘ providing a communicator unit (122) with communicator electronics,
∘ coupling the adapter module (123) to the communicator module and electrically connecting the adapter electronics to the communicator electronics (124),
- electrically connecting the communicator electronics to the motor head electronics (130).

## Revendications

1. Bioréacteur (1), en particulier destiné à la culture de micro-organismes et/ou de cultures cellulaires, comprenant
- un récipient (13),
- une plaque supérieure (11) conçue pour fermer hermétiquement le récipient (13),
- un mécanisme d'agitation comprenant
∘ un arbre d'agitation (12) guidé à travers la plaque supérieure (11) vers l'intérieur (14) du récipient (13), et
∘ un élément d'agitation relié à l'arbre d'agitation (12) à l'intérieur du récipient (13),
- une unité d'entraînement (15) disposée sur la plaque supérieure (11) et couplée à l'arbre d'agitation (12), et
- au moins un dispositif de terrain (50, 50a, 50b) disposé à l'intérieur (14) et comprenant une électronique de dispositif de terrain,
dans lequel la plaque supérieure (11) comprend :
- une face intérieure et une face extérieure opposée à la face intérieure,
- un dispositif de raccordement de l'agitateur configuré pour
∘ guider l'arbre d'agitation (12) à travers la plaque de tête (11) et
∘ raccorder l'unité d'entraînement (15),
- au moins un moyen de raccordement de dispositif de terrain configuré pour raccorder le dispositif de terrain (50, 50a, 50b) à la face interne, et
- une unité de transmission de données disposée sur le côté extérieur, comprenant
∘ un ensemble de tête de moteur (40) qui est couplé par signal à une unité d'évaluation et couplé en puissance à l'unité d'entraînement (15), comprenant une électronique de tête de moteur, et
∘ au moins une unité de communication couplée électriquement à l'unité de tête de moteur (40), comprenant :
▪ une unité d'adaptation (20, 20a, 20b) avec une électronique d'adaptation qui est connectée électriquement à l'électronique du dispositif de terrain (50, 50a, 50b), et
▪ une unité de communication (30, 30a, 30b) avec une électronique de communication qui est connectée électriquement à l'électronique d'adaptateur et à l'électronique de la tête de moteur,
dans lequel l'électronique d'adaptateur est configurée pour transmettre des signaux entre l'électronique du dispositif de terrain et l'électronique de communication, dans lequel les signaux transportent des informations relatives au dispositif de terrain ainsi que des informations de mesure et/ou des informations de commande,
dans lequel l'électronique de communication est configurée pour transmettre les signaux entre l'électronique d'adaptation et l'électronique de la tête de moteur,
dans lequel l'unité de transmission de données comprend en outre un convertisseur de signaux, qui est de préférence réalisé en tant que partie de l'électronique d'adaptateur et/ou en tant que partie de l'électronique de communication ;
dans lequel l'unité d'adaptation (20, 20a, 20b) et l'unité de communication (30, 30a, 30b) sont configurées comme des composants séparés pouvant être interconnectés,
dans lequel la tête de moteur (40) est configurée comme un composant séparé pouvant être couplé à l'unité de communication.

2. Plaque de tête (11) destinée à obturer un bioréacteur (1), comprenant
- une face intérieure et une face extérieure opposée à la face intérieure,
- un dispositif de raccordement d'agitateur configuré pour
∘ guider un arbre d'agitateur (12) à travers la plaque de tête (11) et
∘ raccorder une unité d'entraînement (15) pouvant être couplée à l'arbre d'agitateur (12),
- au moins un moyen de raccordement de dispositif de terrain configuré pour raccorder un dispositif de terrain (50, 50a, 50b) à la face intérieure,
- une unité de transmission de données disposée à l'extérieur, comprenant
∘ un ensemble de tête de moteur (40) pouvant être couplé par signal à une unité d'évaluation et par alimentation à l'unité d'entraînement (15), qui peut être disposé sur une plaque de tête (11) d'un bioréacteur (1), comprenant l'électronique de la tête de moteur, et
∘ au moins une unité de communication couplée électriquement à l'unité de tête de moteur (40), comprenant :
▪ une unité d'adaptation (20, 20a, 20b) avec une électronique d'adaptation pouvant être connectée électriquement à l'électronique du dispositif de terrain (50, 50a, 50b), et
▪ une unité de communication (30, 30a, 30b) avec une électronique de communication qui est reliée électriquement à l'électronique d'adaptateur et à l'électronique de la tête de moteur,
dans lequel l'électronique d'adaptateur est configurée pour transmettre des signaux entre l'électronique du dispositif de terrain et l'électronique de communication, dans lequel les signaux transportent des informations relatives au dispositif de terrain ainsi que des informations de mesure et/ou des informations de commande,
dans lequel l'électronique de communication est configurée pour transmettre les signaux entre l'électronique d'adaptation et l'électronique de la tête de moteur,
dans lequel l'unité de transmission de données comprend en outre un convertisseur de signaux, qui est de préférence intégré à l'électronique de l'adaptateur et/ou à l'électronique du communicateur,
dans lequel l'unité d'adaptation (20, 20a, 20b) et l'unité de communication (30, 30a, 30b) sont configurées comme des composants séparés pouvant être interconnectés,
dans lequel la tête de moteur (40) est configurée comme un composant séparé pouvant être couplé à l'unité de communication.

3. Unité de transmission de données pour une plaque supérieure (11) d'un bioréacteur (1) comprenant
- une tête de moteur (40) pouvant être couplée, sur le plan des signaux, à une unité d'évaluation et, sur le plan de l'alimentation, à une unité d'entraînement (15) pouvant être disposée sur une plaque de tête (11) d'un bioréacteur (1), comprenant une électronique de tête de moteur, et
- au moins une unité de communication couplée à l'ensemble de tête de moteur (40) par transmission de signaux, chacune comprenant :
∘ une unité d'adaptation (20, 20a, 20b) avec une électronique d'adaptation pouvant être connectée électriquement à l'électronique d'un dispositif de terrain (50, 50a, 50b), et
∘ une unité de communication (30, 30a, 30b) avec une électronique de communication qui est connectée électriquement à l'électronique d'adaptateur et à l'électronique de la tête de moteur,
dans lequel l'électronique d'adaptateur est configurée pour transmettre des signaux entre l'électronique du dispositif de terrain et l'électronique de communication, dans lequel les signaux transportent des informations sur le dispositif de terrain et des informations de mesure et/ou des informations de commande,
dans lequel l'électronique de communication est configurée pour transmettre les signaux entre l'électronique d'adaptation et l'électronique de la tête de moteur,
dans lequel l'unité de transmission de données comprend en outre un convertisseur de signaux, qui est de préférence réalisé en tant que partie de l'électronique d'adaptateur et/ou en tant que partie de l'électronique de communication,
dans lequel l'unité d'adaptation (20, 20a, 20b) et l'unité de communication (30, 30a, 30b) sont configurées comme des composants distincts pouvant être couplés l'un à l'autre,
dans lequel la pièce de tête de moteur (40) est configurée comme un composant séparé pouvant être couplé aux unités de communication.

4. Unité de transmission de données selon au moins la revendication 3, dans laquelle
- l'unité d'adaptation (20, 20a, 20b) comprend une interface de dispositif de terrain et une interface de communicateur, l'électronique d'adaptation
∘ est connectée entre l'interface de dispositif de terrain et l'interface de communication,
∘ peut être connectée électriquement à l'électronique de l'appareil de terrain via l'interface de l'appareil de terrain, et
∘ est connectée électriquement à l'électronique du communicateur via l'interface de communicateur, et
- l'unité de communication (30, 30a, 30b) comprend une interface d'adaptateur et une interface de tête de moteur, l'électronique de communication
∘ est connectée entre l'interface d'adaptateur et l'interface de tête de moteur,
∘ est reliée électriquement à l'électronique de l'adaptateur via l'interface d'adaptateur, et
∘ est reliée électriquement à l'électronique de la tête de moteur via l'interface de la tête de moteur.

5. Unité de transmission de données selon au moins l'une des revendications 3 ou 4, comprenant au moins un premier groupe de premières pièces d'adaptation et un deuxième groupe de deuxièmes pièces d'adaptation,
dans laquelle les circuits électroniques d'adaptateur des premières pièces d'adaptateur (20a) sont configurés pour transmettre les signaux entre les circuits électroniques d'un premier dispositif de terrain (50a) et les circuits électroniques du communicateur,
dans lequel les circuits électroniques d'adaptation des deuxièmes éléments d'adaptation (20b) sont configurés pour transmettre les signaux entre les circuits électroniques d'un deuxième dispositif de terrain (50b) et les circuits électroniques du communicateur,
dans lequel le module adaptateur (20, 20a, 20b) de chaque unité de communication est sélectionné, en fonction d'un dispositif de terrain (50, 50a, 50b) à coupler, parmi au moins le premier groupe de premiers modules adaptateurs (50a) et le deuxième groupe de deuxièmes modules adaptateurs (50b).

6. Unité de transmission de données selon au moins l'une des revendications 3 à 5, dans laquelle
- la pièce d'adaptation (20, 20a, 20b) comprend un dispositif de couplage de dispositif de terrain configuré pour relier mécaniquement le dispositif de terrain (50, 50a, 50b) à la pièce d'adaptation (20, 20a, 20b), de préférence de manière amovible, et/ou
- la pièce de communication (30, 30a, 30b) comprend un raccord d'adaptateur configuré pour relier mécaniquement la pièce d'adaptateur (20, 20a, 20b) à la pièce de communication (30, 30a, 30b), de préférence de manière amovible.

7. Unité de transmission de données selon au moins l'une des revendications 3 à 6,
- dans laquelle le convertisseur de signal est un convertisseur analogique-numérique,
et/ou
- dans laquelle l'électronique de l'adaptateur et/ou l'électronique du communicateur et/ou le convertisseur de signaux est configurée pour recevoir des signaux analogiques, les convertir en signaux analogiques normalisés, convertir les signaux analogiques normalisés en signaux numériques, et transmettre les signaux numériques, et/ou inversement.

8. Unité de transmission de données selon au moins l'une des revendications 3 à 7, dans laquelle l'électronique du communicateur et/ou l'électronique de la culasse sont configurées pour recevoir les signaux sans contact ou via une connexion, de préférence via un câble, pour convertir les signaux en signaux radio, et pour les transmettre par radio ou via un câble et/ou inversement.

9. Unité de transmission de données selon au moins l'une des revendications 3 à 8, dans laquelle l'électronique de l'adaptateur est configurée pour recevoir les signaux sans contact ou via une connexion physique, de préférence via un câble, pour convertir les signaux en signaux radio, et pour les transmettre par radio ou via un câble et/ou inversement.

10. Unité de transmission de données selon au moins la revendication 4 et l'une des revendications 5 à 9, dans laquelle l'interface du dispositif de terrain et/ou l'interface du communicateur et/ou l'interface de l'adaptateur et/ou l'interface de la tête de moteur est configurée comme un connecteur sur un câble, le câble ayant de préférence une longueur maximale de 50 cm.

11. Unité de transmission de données selon au moins l'une des revendications 3 à 10, dans laquelle
- l'unité de tête de moteur (40) est couplée électriquement à l'unité de communication (30, 30a, 30b), l'électronique de la tête de moteur étant configurée pour alimenter l'électronique de communication, et/ou
- l'unité de communication (30, 30a, 30b) est couplée électriquement à l'unité d'adaptation (20, 20a, 20b), l'électronique de l'unité de communication étant configurée pour alimenter l'électronique de l'unité d'adaptation, et/ou
- l'unité d'adaptation (20, 20a, 20b) est connectable électriquement au dispositif de terrain (50, 50a, 50b), l'électronique de l'unité d'adaptation étant configurée pour alimenter l'électronique du dispositif de terrain,
l'alimentation étant de préférence transmise via une connexion par contact, de préférence via un câble, et/ou par induction.

12. Unité de transmission de données selon au moins l'une des revendications 3 à 11, dans laquelle la tête de moteur (40) comprend :
- une interface de communication qui est connectée électriquement à l'électronique du communicateur, et
- une interface de ligne d'alimentation à laquelle un câble de ligne d'alimentation peut être connecté électriquement, dans laquelle l'interface de ligne d'alimentation peut être connectée électriquement à l'électronique d'évaluation de l'unité d'évaluation,
dans laquelle l'électronique de la tête de moteur est connectée entre l'interface de communication et l'interface de ligne d'alimentation,
dans laquelle l'électronique de la tête de moteur est configurée pour transmettre des signaux entre l'électronique du communicateur, qui est connectée électriquement à l'interface de communication, et l'électronique d'évaluation qui peut être connectée électriquement à l'interface de ligne d'alimentation via un système de bus ou plusieurs systèmes de bus ( ).

13. Unité de transmission de données selon au moins l'une des revendications 3 à 12, dans laquelle l'électronique de la tête de moteur est configurée pour transmettre des signaux basés sur un protocole de bus via un ou plusieurs systèmes de bus à l'électronique de communication des unités de communication (30, 30a, 30b) via un système de bus ou plusieurs systèmes de bus, ou pour recevoir de tels signaux provenant de l'électronique de communication des éléments de communication (30, 30a, 30b).

14. Utilisation d'une unité de transmission de données selon l'une quelconque des revendications 3 à 13 pour une plaque de tête (11), en particulier selon au moins la revendication 2, et/ou pour un bioréacteur (1), en particulier selon au moins la revendication 1.

15. Procédé pour fournir une unité de transmission de données selon l'une quelconque des revendications 3 à 13, comprenant les étapes suivantes :
- fournir une tête de moteur (110) pouvant être couplée, sur le plan des signaux, à une unité d'évaluation et, sur le plan de l'alimentation, à une unité d'entraînement, qui peut être disposée sur une plaque de tête d'un bioréacteur, comprenant une électronique de tête de moteur, et
- assembler au moins une unité de communication (120), comprenant les étapes suivantes :
∘ sélectionner une unité d'adaptation (121) dotée d'une électronique d'adaptation en fonction du dispositif de terrain à coupler à cette unité d'adaptation,
∘ fournir une unité de communication (122) avec une électronique de communication,
∘ raccorder le module adaptateur (123) au module communicateur et relier électriquement l'électronique d'adaptateur à l'électronique de communicateur (124),
- connecter électriquement l'électronique de communication à l'électronique de la tête de moteur (130).
